# EUROPÄISCHE PATENTSCHRIFT

(11) **EP 0 557 877 B1**
(45) Veröffentlichungstag und Bekanntmachung des Hinweises auf die Patenterteilung: **24.09.1997**
(21) Anmeldenummer: 93102451.7
(22) Anmeldetag: 17.02.1993
(51) Int. Cl.: C07D 403/04, C07D 233/38, A61K 31/505

(54) **Lösliche Salze von 4-Amino-2-(4,4-dimethyl-imidazolidin-2-on-1-yl)-pyrimidin-5 -carbonsäure- N-methyl-N-(3-trifluormethyl-phenyl)- amid, Verfahren zu ihrer Herstellung, ihre Verwendung als Arzneimittel und Ausgangsprodukte**
Soluble salts of 4-amino-2-(4,4-dimethyl-imidazolidin-2-on-1-yl)-pyrimidin-5-carboxylic acid N-methyl-N-(3-trifluoromethyl-phenyl)-amide, process for their preparation, their use as medicine and initial products
Sels solubles d'acide 4-amino-2-(4,4-diméthyl-imidazolidin-2-on-1-yl)-5-pyrimidine-carboxylique-N-méthyl-N-(3-trifluorométhyl-phényl)-amide, procédé pour leur préparation, leur usage comme médicament et produits de départ

(30) Priorität: 22.02.1992 DE 4205483
(43) Veröffentlichungstag der Anmeldung: 01.09.1993
(73) Patentinhaber: HOECHST AKTIENGESELLSCHAFT, 65926 Frankfurt am Main (DE)
(72) Erfinder: Kampe, Klaus-Dieter, Dr., W-6232 Bad Soden/Ts. (DE); Granzer, Ernold, Dr. Dr., W-6233 Kelkheim/Ts. (DE); Leineweber, Michael, Dr., W-6230 Frankfurt/M (DE); Hüttinger, Manfred, Prof. Dr., A-1232 Wien (AT)

(56) Entgegenhaltungen:
- EP-A- 0 012 361
- EP-A- 0 206 297

## Beschreibung

Die Erfindung betrifft das in Wasser leicht lösliche Hydrochlorid von 4-Amino-2-(4,4-dimethyl-imidazolidin-2-on-1-yl)-pyrimidin-5-carbonsäure-[N-methyl-N-(3-trifluormethyl-phenyl)-amid].

Es ist bereits beschrieben worden, 4-Amino-2-ureido-pyrimidin-5-carbonsäure-(N-phenyl-amide) zur Behandlung von Adipositas und von Lipidstoffwechselstörungen [vgl. Europ. Patentschrift B-0 012 361 (US-Patent Nr. 4 285 946) zu verwenden. Außerdem ist beschrieben worden, eine - bezüglich ihrer Struktur spezielle - Gruppe dieser Verbindungen, nämlich entsprechende N-(3-Trifluormethyl-phenyl)-amide, zur Prophylaxe gegen und zur Behandlung von Thrombosen anzuwenden [vgl. Europ. Patentschrift B-0 206 297 (US-Patent Nr. 4 705 792)].

In gewisser Weise nachteilig wirkt sich bei der Verwendung dieser Verbindungen als Arzneimittel deren Schwerlöslichkeit in Wasser sowie in physiologisch tolerablen Lösemitteln bzw. Lösemittelmischungen, inklusive Mischungen von solchen Lösemitteln mit Wasser, aus. Dieser Nachteil ist insbesondere bei den bevorzugten, den Lipidstoffwechsel sehr günstig beeinflussenden Verbindungen gemäß EP-B-0 012 361 und bei stark antithrombotisch wirksamen Verbindungen (vgl. EP-B-0 206 297) beobachtet worden.

Es wurde nun überraschend gefunden, daß 4-Amino-2-(4,4-dimethyl-imidazolidin-2-on-1-yl)-pyrimidin-5-carbonsäure-[N-methyl-N-(3-trifluormethyl-phenyl)-amid] der Formel I 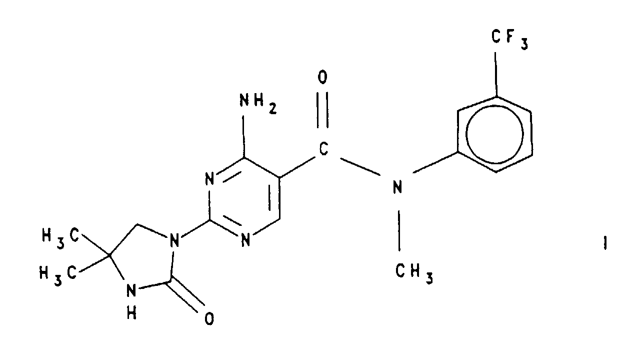 in Form des Hydrochlorids in Wasser leicht löslich ist. Die Erfindung betrifft daher das in Wasser leicht lösliche physiologisch verträgliche Hydrochlorid der Verbindung der Formel I.

Als in Wasser leicht lösliche Salze sind gemäß der Definition im "Deutschen Arzneibuch" (9. Ausgabe 1986, Amtliche Ausgabe, Deutscher Apotheker-Verlag Stuttgart), Seite 19, solche zu verstehen, bei denen sich ein Masseteil des betreffenden Salzes der Verbindung I in 1-10 Masseteilen (= Volumenteilen) Wasser löst. Als lösliche Salze sind gemäß vorgenannter DAB-Definition diejenigen zu verstehen, bei denen sich ein Masseteil des betreffenden Salzes der Verbindung I in 10-30 Masseteilen Wasser löst.

In den folgenden Ausführungen umfaßt der Ausdruck "leicht lösliche Salze" auch "lösliche" Salze gemäß dieser Definition, sofern nichts anderes angegeben ist.

Diese sehr vorteilhafte Eigenschaft, die gute Löslichkeit in Wasser des Hydrochlorids der vorgenannten Verbindung I, ist ausgesprochen überraschend, da sowohl Salze wie auch die freien Basen analog aufgebauter Verbindungen, die keine Methylgruppe am Amid-Stickstoffatom tragen, in Wasser ausgesprochen schwerlöslich sind. So ist beispielsweise vom Hydrochlorid des 4-Amino-2-(4,4-dimethyl-imidazolidin-2-on-1-yl)-pyrimidin-5-carbonsäure-[N-(3-trifluormethyl-phenyl)-amids] in Wasser bei 23°C nur eine 0,93x10⁻³ molare Lösung herstellbar, was bedeutet, daß sich 1 Masseteil dieser Verbindung in 2500 Teilen Wasser löst.

Verbindung I in Form der freien Base ist, wie auch 4-Amino-2-(4,4-dimethylimidazolidin-2-on-1-yl)-pyrimidin-5-carbonsäure-[N-(3-trifluormethyl-phenyl)-amid] in Form der freien Base ebenfalls in Wasser ausgesprochen schwerlöslich.

Aufgrund dieser Eigenschaften und der allgemein bei den in der Europäischen Patentschrift 0 012 361 beschriebenen Verbindungen festzustellenden Schwerlöslichkeit sowohl der freien Verbindungen wie auch der Salze in Wasser, war keinesfalls zu erwarten, daß das Hydrochlorid der N-Methyl-Verbindung I in Wasser leicht löslich ist.

Die sprunghafte Verbesserung der Löslichkeit von Salzen der Verbindung I in Wasser ist auch insofern überraschend, da die strukturelle Besonderheit der Verbindung I, nämlich der Ersatz des Wasserstoffatoms am Amid-Stickstoffatom durch eine Methylgruppe, in der Einführung eines gegenüber Wasserstoff lipophileren Restes besteht. Mit diesem Austausch - Wasserstoff gegen Methyl- verschwindet in der Struktur eine potentielle Wasserstoffbrückenbindung. Die Struktur der Verbindung I sollte demzufolge weniger polar werden. Das wiederum sollte keinesfalls zu einer besseren Löslichkeit der Salze in Wasser führen. Bekanntlich zeigen sekundäre Amidgruppen (mit der Struktureinheit HN-CO) eine große Tendenz zur Ausbildung von Wasserstoffbrückenbindungen, die in der Regel zu polaren und infolge dessen tendenziell hydrophileren Stoffen führen. Bei der Verbindung I tritt gegenüber analogen Verbindungen mit einer sekundären Amidgruppe überraschenderweise der umgekehrte Effekt ein, indem hier bei der eine tertiäre Amidgruppe enthaltenden Verbindung I durch Salzbildung ausgeprägt hydrophile Stoffe entstehen.

Die gute Wasserlöslichkeit des Hydrochlorids der Verbindung I bringt einige bedeutende Vorteile für die Verwendung als Arzneimittel. Das in Wasser leicht lösliche Hydrochlorid der Verbindung I wirkt sich aufgrund dieser Eigenschaft günstig auf die Resorption dieses Wirkstoffs durch den Organismus des zu behandelnden Individuums aus. Demzufolge ergibt sich auch eine günstige Auswirkung auf die Bioverfügbarkeit des Wirkstoffs in dem betreffenden Organismus.

Vorteile bringt die gute Wasserlöslichkeit insbesondere auch für die galenischen Zubereitungen dieses Wirkstoffs. Bekanntlich sind Wirkstoffe, die leicht wasserlösliche Salze bilden, galenisch einfacher und sicherer zu handhaben bzw. zu bearbeiten als schwerlösliche oder nahezu wasserunlösliche Verbindungen. Insgesamt gesehen vereinfachen sich viele Probleme, die die Zubereitung und die Anwendung der Verbindung I als Arzneistoff betreffen. Darüber hinaus ist ein wasserlöslicher Arzneistoff, wie das gut in Wasser lösliche Monohydrochlorid der Verbindung I, beispielsweise aucn bezüglich der Dosierung leichter und sicherer anzuwenden.

In der nachstehenden Tabelle 1 sind zum Zweck des Vergleichs Löslichkeiten in Wasser von Salzen ähnlicher, am Amid-Stickstoffatom nicht methylierter Verbindungen aufgeführt. Wie aus der Tabelle 1 ersichtlich, handelt es sich bei den aufgeführten N-Desmethyl-Verbindungen (Nr. 1-11) mit unterschiedlichen Substitutionsmustern bis auf die wenig in Wasser lösliche Verbindung Nr. 6 durchweg um schwer und sehr schwer in Wasser lösliche Salze.

In der Tabelle 2 ist die Löslichkeit des in Wasser dagegen leicht löslichen erfindungsgemäßen Monohydrochlorids der Verbindung I wiedergegeben.

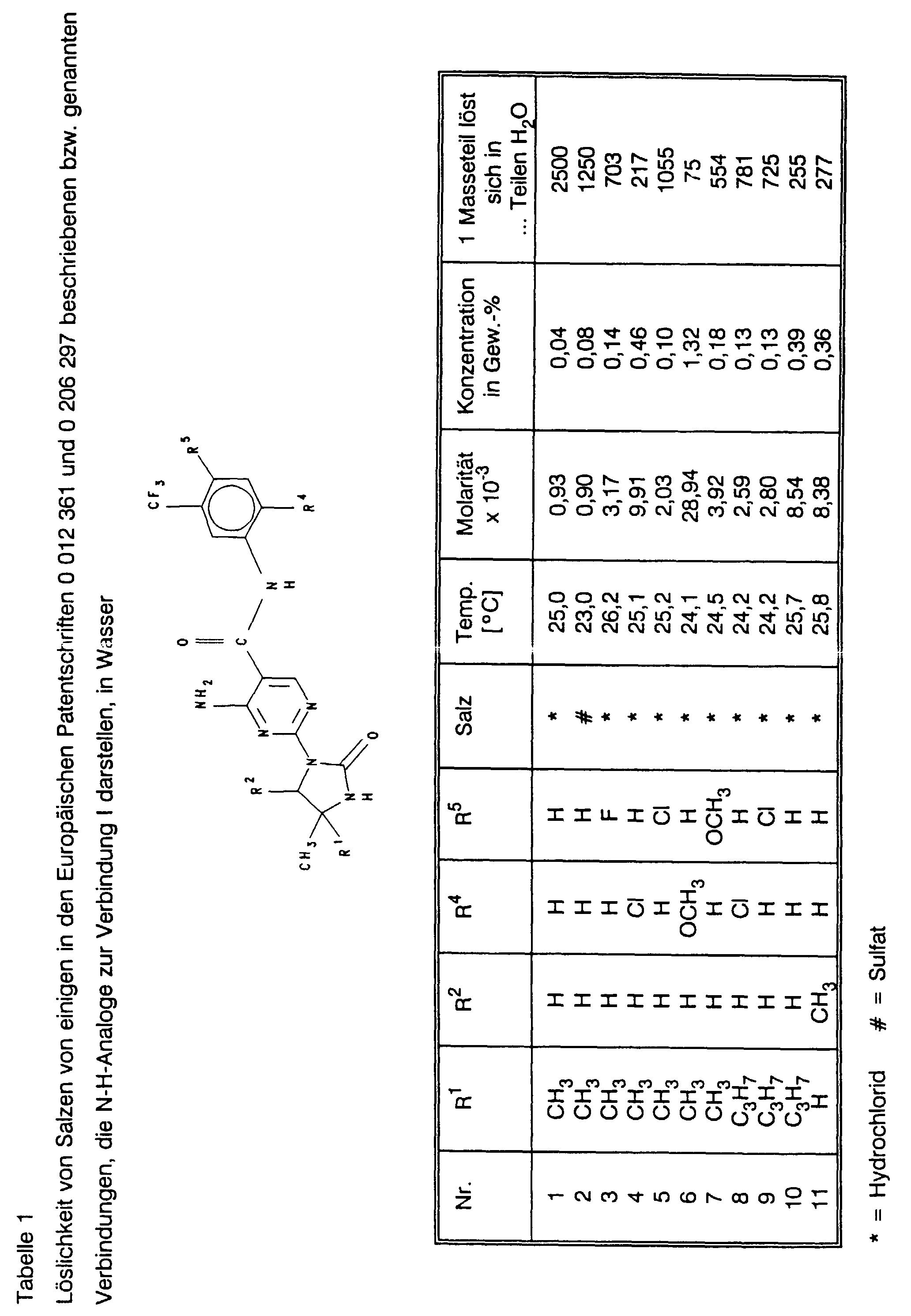

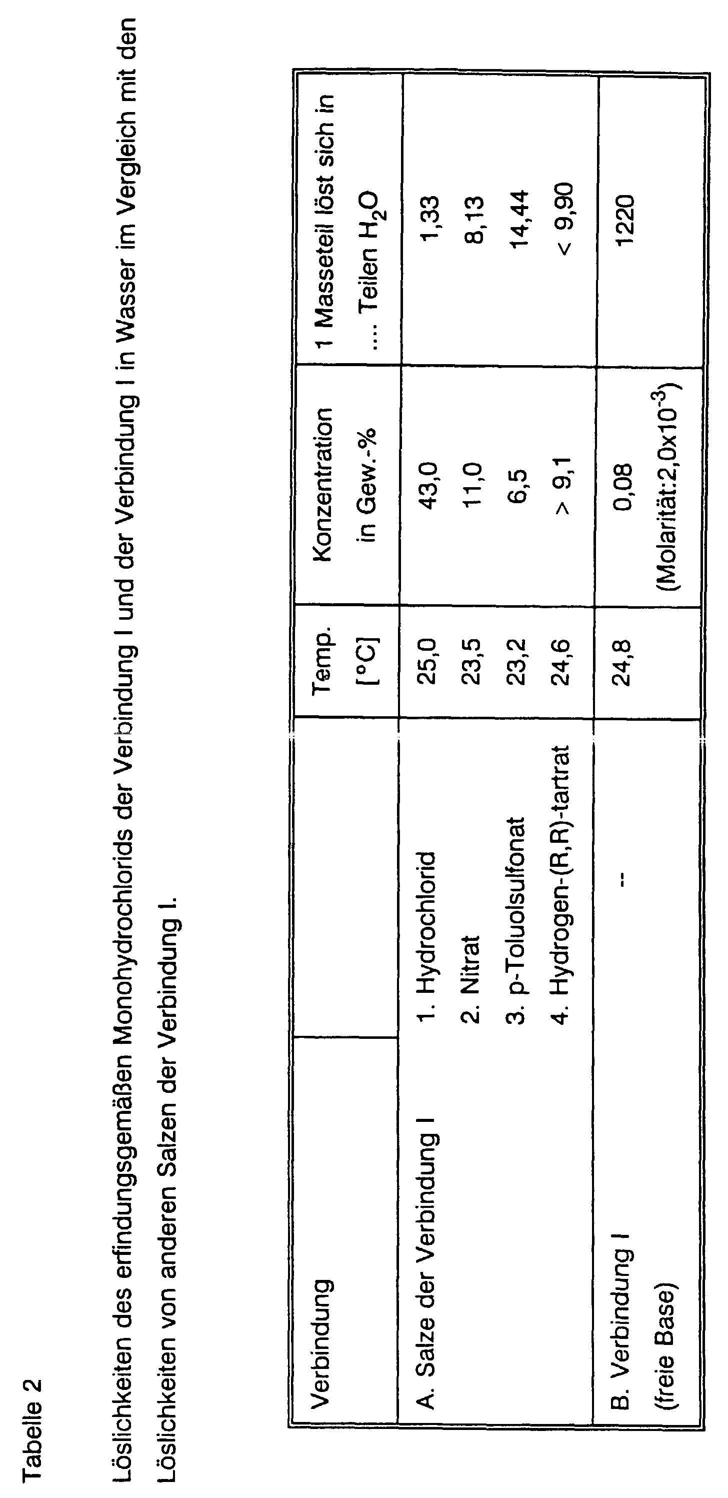

Die Erfindung betrifft weiterhin ein neues Verfahren zur Herstellung des Hydrochlorids von 4-Amino-2-(4,4-dimethyl-imidazolidin-2-on-1-yl)-pyrimidin-5-carbonsäure-[N-methyl-N-(3-trifluormethyl-phenyl)-amid] der Formel I, dadurch gekennzeichnet, daß man eine Verbindung der Formel IV 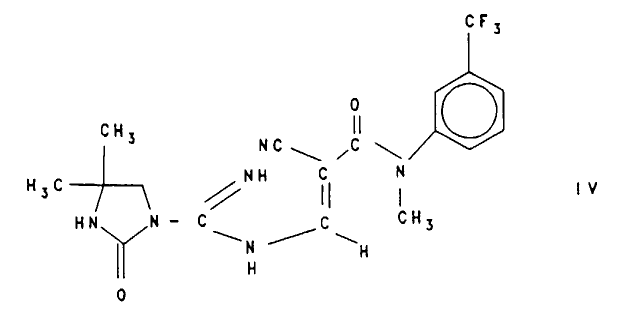 oder ein Salz oder ein Salzgemisch dieser Verbindung bei einer Temperatur von 0° bis 240°C mit oder ohne Lösemittelzusatz zu der Verbindung der Formel I oder einem Salz oder einem Salzgemisch dieser Verbindung cyclisiert, eine erhaltene Verbindung der Formel I in das Hydrochlorid überführt oder ein erhaltenes Salz oder Salzgemisch in das Hydrochlorid überführt.

Das Guanidin-Derivat der Formel IV ist eine neue Verbindung. Die Erfindung betrifft daher auch die Verbindung der Formel IV in Form von beiden möglichen Stereoisomeren, der E- und der Z-Form, sowie deren Säureadditionssalze.

Die Erfindung betrifft ferner ein Verfahren zur Herstellung der Salze der Verbindung der Formel IV, das dadurch gekennzeichnet ist, daß man eine Verbindung der Formel II 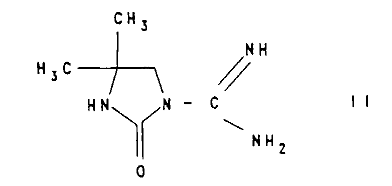 oder ein Salz dieser Verbindung mit einer Verbindung der Formel III 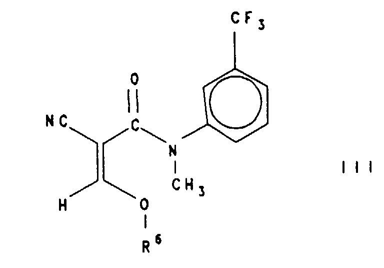 worin R⁶ C₁-C₄-Alkyl bedeutet, in Gegenwart eines organischen Löse- oder Verdünnungsmittels unter Bildung der Verbindung der Formel IV oder einem Salz dieser Verbindung umsetzt, und die erhaltene Verbindung der Formel IV in ein Salz überführt.

Die Verbindung IV fällt in der Regel als Stereoisomereinmischung (E und Z) an. Sie kann aber auch als reines Stereoisomeres (E- oder Z-Form) gebildet werden. Die Verbindung IV bzw. deren Salze dienen als Ausgangsprodukt zur Herstellung der Verbindung I bzw. deren löslichen sowie leicht wasserlöslichen Salzen. Die Verbindung IV bildet als einsäurige Base Säureadditionssalze. Für deren Bildung sind im Prinzip alle Protonensäuren geeignet; vorteilhaft werden starke bis mittelstarke Säuren zur Salzbildung herangezogen. Als Beispiele seien folgende genannt: Chlor-, Brom- oder Jodwasserstoffsäure, Schwefel-, Phosphor-, Salpeter- oder Perchlorsäure, (C₁-C₄)-Alkan-phosphonsäuren, Sulfonsäuren wie Methan-, Benzol-, Toluol- oder Trifluormethan-sulfonsäure, Sulfaminsäure, Methylschwefelsäure, Essig-, Chloressig-, Dichloressig-, Trichloressig- oder Trifluoressigsäure, Ameisen-, Propion-, Oxal-, Malon-, Malein-, Bernstein-, Glutar-, Äpfel-, Wein-, Zitronen-, Fumar-, Milch-, Glykol- oder Brenztraubensäure, Benzoe- oder im Phenylrest substituierte Benzoesäuren wie Toluylsäure oder p-Nitro-benzoesäure oder Salicylsäure, Furancarbon- und/oder Mandelsäure.

Die als Ausgangsstoff zur Herstellung der Verbindung IV erforderliche Verbindung II ist bekannt. Die Verbindungen der Formel III können nach für diesen Enolether-Typ bekannten Methoden, ausgehend von dem bekannten 3-Methylaminobenzotrifluorid, hergestellt werden (Angew. Chemie Suppl. 1982, 1213).

Für den Fall, daß die Verbindung II in Form eines Salzes angewandt wird, wird eine, vorteilhaft äquimolare, Menge einer basischen Verbindung bei der Umsetzung mit einer Verbindung der Formel III zusätzlich angewendet.

Unter einem Salz der Verbindung II sind mit anorganischen sowie mit organischen Säuren zu erhaltende Säureadditionssalze zu verstehen. Bevorzugte Salze der Verbindung II sind das Hydrobromid, Hydrochlorid oder das Sulfat. Besonders geeignet für das erfindungsgemäße Verfahren sind das Hydrobromid und/oder -chlorid.

Die Umsetzung des Amidino-imidazolidinons der Formel II mit einer Verbindung der Formel III kann unter unterschiedlichsten Bedingungen erfolgen. Das betrifft sowohl die Reaktionstemperatur wie auch gegebenenfalls mitzuverwendende Löse- bzw. Verdünnungsmittel. So kann die erfindungsgemäße Umsetzung der Verbindung II mit einer Verbindung III bei einer Temperatur von -100°C bis +200°C mit oder ohne Zusatz eines Löse- bzw. Verdünnungsmittels erfolgen. Zweckmäßig wird diese Umsetzung von -30°C, vorzugsweise +10°C, bis +100°C, vorzugsweise +35°C, in Gegenwart von Löse- bzw. Verdünnungsmitteln durchgeführt.

Als Löse- bzw. Verdünnungsmittel sind im Prinzip alle gegenüber den Verbindungen II und III (weitgehend) inerten Lösemittel, inklusive Wasser, brauchbar. Vorteilhaft werden C₁-C₅-Alkohole, Tetrahydrofuran, Dioxan, niedere aliphatische Ether, wie Diethyl-, Methyl-t.-butyl- sowie Diisopropylether, Acetonitril, N,N-Dimethylformamid, N,N-Dimethylacetamid, niedere Glykol- und Diglykol-di- und -monoether, wie 1,2-Dimethoxyethan, Methylglykol, Ethylglykol, Diglykoldimethyl- und -monomethylether, Ethylacetat, Methylacetat, Toluol, Pyridin, Aceton, Chloroform und/oder Dichlormethan als Löse- bzw. Verdünnungsmittel verwendet.

Bevorzugte Löse- bzw. Verdünnungsmittel sind C₂-C₄-Alkohole, Tetrahydrofuran, Dioxan, 1,2-Dimethoxyethan, Methyl-t.-butylether und/oder Acetonitril. Es können auch Mischungen der aufgeführten Lösungsmittel verwendet werden, sowie auch Mischungen der aufgeführten mit anderen Lösungsmitteln.

Bei der erfindungsgemäßen Umsetzung der Verbindung II mit einer Verbindung III können die (stöchiometrischen) Mengenverhältnisse, in denen diese Verbindungen angewandt werden, innerhalb eines breiten Bereichs schwanken. II und III können in äquivalenten oder in nicht-äquivalenten Mengen miteinander umgesetzt werden. Letzteres bedeutet, daß eine der beiden Verbindungen in einem stöchiometrischen Überschuß angewendet werden kann. Bevorzugt wird die Verbindung II als Base mit III in äquivalenten Mengen oder in einem Verhältnis, wobei die Verbindung III in einem stöchiometrischen Überschuß vorliegt, umgesetzt. Wird die Verbindung II als Säureadditionssalz angewandt, so kann auch sie gegenüber III in einem stöchiometrischen Überschuß eingesetzt werden.

Die erforderlichen Reaktionszeiten sind von der Temperatur abhängig, bei der die Umsetzung durchgeführt wird. Sie können innerhalb eines breiten Bereichs schwanken. In der Regel betragen die Reaktionszeiten, wenn Temperaturen von +10°C bis +60°C angewandt werden, 0,3-10 Stunden, wobei die Reaktionszeiten - wie bekannt - im umgekehrten Verhältnis zur Temperatur stehen.

Wird die Verbindung II in Form eines Salzes angewandt, dann wird bei der erfindungsgemäßen Umsetzung - zweckmäßig in equimolarer Menge - eine basische Verbindung zugesetzt, bzw. die basische Verbindung II wird, bevor die Zugabe einer Verbindung III erfolgt, mit einer basischen Verbindung aus ihrem Salz teilweise oder vollständig freigesetzt. Bevorzugt läßt man die zu diesem Zweck mitverwendete basische Verbindung vor Zugabe einer Verbindung der Formel III auf das angewandte Salz des Amidino-imidazolidinons II einwirken. Die erfindungsgemäße Umsetzung kann somit auch in dieser Ausführungsform als "Eintopfreaktion" durchgeführt werden. Als basische Zusätze können anorganische und/oder organische basische Verbindungen verwendet werden. Als Beispiele seien genannt: Alkali- oder Erdalkalialkoholate von niederen Alkoholen, Alkali- oder Erdalkalihydroxide oder -hydride, als rein organische Basen tertiäre Amine, wie niedere Trialkylamine und/oder N,N-Dimethylanilin, Diazabicycloundecen (DBU) sowie analoge cyclische (trisubstituierte) Amidine, Tetraalkylammoniumhydroxide, 2- oder 4-Dialkylamino-pyridine und/oder Diazabicyclo-[2.2.2]-octan (Dabco).

Vorzugsweise werden Alkalialkoholate von (C₁-C₃)-Alkanolen, Natriumhydrid, DBU und DBU-analoge Amidine oder Tetramethyl-ammonium-hydroxid als basische Zusätze verwendet, wobei Natrium- oder Kaliummethylat und/oder -ethylat besonders geeignet sind.

Eine vorteilhafte Ausführungsform des erfindungsgemäßen Verfahrens besteht daher darin, daß die Verbindung der Formel II in gelöster Form bei einer Temperatur von 10°C bis 35°C mit einer Verbindung der Formel III, in der R⁶ die oben angegebene Bedeutung hat, wobei R⁶ bevorzugt Methyl oder Ethyl bedeutet, umgesetzt wird. Nach einer Reaktionszeit von 0,3-4 Stunden wird die gebildete, in der Regel als schwer lösliches Kristallisat abgeschiedene Verbindung der Formel IV durch Abfiltrieren isoliert und bei einer Temperatur von 10°C bis 35°C - zweckmäßig unter Vakuum - getrocknet.

In Kenntnis dessen, was aus den eingangs zitierten europäischen Patentschriften bekannt ist, war es überraschend, daß bei der Umsetzung von Verbindung II mit einer Verbindung III die Verbindung IV als kristalliner Feststoff in hohen Ausbeuten und hoher Reinheit isoliert werden kann.

Bei der Herstellung der in der europäischen Patentschrift 0 012 361 beschriebenen Verbindungen sind Verbindungen vom Typ der Verbindung IV nicht beobachtet worden. Insbesondere ist die Stabilität der Verbindung IV überraschend, die eine bequeme Isolierung dieser als Ausgangsstoff für das erfindungsgemäße Verfahren erforderlichen Verbindung in guten Ausbeuten ermöglicht.

Die Isolierung der Verbindung der Formel IV ist leicht möglich aufgrund der generellen Schwerlöslichkeit dieser Verbindung, insbesondere in für das erfindungsgemäße Verfahren geeigneten, organischen Lösemitteln.

Da die Verbindung IV im Rahmen der vorliegenden Erfindung zu dem Endprodukt der Formel I bzw. zu den leicht löslichen Salzen dieser Verbindung I umgesetzt wird, ist es nicht zwingend erforderlich, die Verbindung IV bzw. die Salze in reiner Form zu isolieren. Vielmehr kann die Cyclisierung unter Änderung der Reaktionsbedingungen im Sinne einer Eintopfreaktion durchgeführt werden.

Die Cyclisierung der Verbindung IV als Base kann mit oder ohne Zusatz eines Löse- oder Verdünnungsmittels erfolgen. Die Cyclisierung in Gegenwart von Löse- bzw. Verdünnungsmitteln wird bei einer Temperatur zwischen 0°C, vorzugsweise 40°C, und 130°C, vorzugsweise 95°C, ausgeführt. Die Cyclisierung ohne Löse- bzw. Verdünnungsmittelzusatz wird bei einer Temperatur von 60°C, vorzugsweise 160°C, bis 240°C, vorzugsweise 200°C, durchgeführt.

Wird die Verbindung IV als Base unter Bildung der Verbindung I cyclisiert, dann besteht eine Ausführungsform darin, daß man die zweckmäßigerweise trockene Verbindung der Formel IV auf eine Temperatur von 160°C bis 200°C 3-27 Minuten - vorzugsweise 10-20 Minuten - lang erhitzt und danach sofort auf Raumtemperatur abkühlt. Anschließend wird die bei der thermischen Cyclisierung aus IV gebildete Verbindung I isoliert. Das beinhaltet in der Regel die Abtrennung von zwei in spürbaren Mengen mitentstandenen Nebenprodukten, dem 3-Methylaminobenzotrifluorid (Formel V im Reaktionsschema I) und der Verbindung der Formel VI (vgl. Schema I). Die Verbindung VI kann leicht aufgrund ihrer Löslichkeit in verdünnten wäßrigen Alkalien und die Verbindung der Formel V größtenteils aufgrund ihrer größeren Flüchtigkeit destillativ von der Verbindung I abgetrennt werden. Die vollständige Abtrennung der Verbindung V von der Verbindung I ist einfach möglich durch Umkristallisieren und/oder durch Auswaschen der kristallinen Verbindung I mit solchen Lösemitteln, die die Verbindung I schwer und die Verbindung V leicht lösen.

Die Cyclisierung der Verbindung IV in Form eines Säureadditionssalzes wird vorzugsweise unter Mitverwendung von Löse- bzw. Verdünnungsmitteln bei einer Temperatur von 0°C, vorzugsweise 45°C, bis 150°C, vorzugsweise 95°C, ausgeführt. Diese Cyclisierung eines Salzes der Verbindung IV kann aber auch ohne Zusatz eines Löse- oder Verdünnungsmittels bei einer Temperatur von 60°C, vorzugsweise 160°C, bis 240°C, vorzugsweise 200°C, ausgeführt werden.

Für diese Art der Cyclisierung der Verbindung IV, mit oder ohne Lösemittelzusatz, können definierte, gesondert aus der Verbindung IV hergestellte Säureadditionssalze sowie auch Mischungen, bestehend aus der Verbindung IV und einer oder mehrerer Säuren verwendet werden. Für solche Mischungen sind alle Zusammensetzungen, beginnend bei minimalsten Mengen an Säureäquivalenten, z.B. kleiner als 10⁻⁴ Äquivalenten, bis zu 2 Säureäquivalenten oder mehr verwendbar; vorzugsweise werden 0,8-1,6 Säureäquivalente angewandt. Im Extremfall kann die Cyclisierung der Verbindung IV in einer Säure, die gleichzeitig als Löse- bzw. Verdünnungsmittel dient, durchgeführt werden. Diese Ausführungsform ist bevorzugt, falls Ameisen-, Essig- und/oder Propionsäure als Säuren und als Verdünnungsmittel verwendet werden. Bei der Cyclisierung von Säureadditionssalzen der Verbindung IV, unter Bildung der Verbindung I, ist also nicht zwingend der Einsatz von definierten, separat hergestellten Salzen erforderlich.

Die Cyclisierung der Verbindung IV unter Zusatz einer oder mehrerer Säuren in Gegenwart von Löse- bzw. Verdünnungsmitteln ist eine gut geeignete Ausführungsform des erfindungsgemäßen Verfahrens.

Eine vorteilhafte Ausführungsform der Cyclisierung von Salzen der Verbindung IV bzw. der Cyclisierung der Verbindung IV unter Zusatz einer oder mehrerer Säuren besteht darin, daß man die Verbindung IV unter Zusatz von 0,2 bis 2,5 oder mehr - vorzugsweise 1 bis 1,5 - Säureäquivalenten in Gegenwart eines Löse- bzw. Verdünnungsmittels auf eine Temperatur von 25°C, vorzugsweise 45°C, bis 120°C, vorzugsweise 95°C, erwärmt. Die Reaktionszeiten betragen dabei, in Abhängigkeit von der Temperatur 0,5-9 Stunden. Anschließend kann durch Zusatz von geeigneten Lösemitteln, worin das entsprechende Salz der gebildeten Verbindung I schwer löslich ist, dieses kristallin abgeschieden werden. Auf diese Weise gelingt eine einfache und effektive Abtrennung der gebildeten Verbindung I von mitentstandenen Nebenprodukten (vgl. dazu das Reaktionsschema I).

Für die vorstehend beschriebene Ausführung der Cyclisierung der Verbindung IV sind im Prinzip alle Protonensäuren brauchbar. Als Beispiele seien folgende genannt: Chlor-, Brom-, Fluor- oder Jodwasserstoffsäure, Schwefel-, Phosphor-, Polyphosphor-, Salpeter- oder Perchlorsäure, (C₁-C₄)-Alkan-phosphonsäuren, Sulfonsäuren wie Methan-, Benzol-, Toluol- oder Trifluormethan-sulfonsäure, Sulfaminsäure, Methylschwefelsäure, Essig-, Cyanessig-, Chloressig-, Dichloressig-, Trichloressig- oder Trifluoressigsäure, Ameisen-, Propion-, Oxal-, Malon-, Malein-, Bernstein-, Glutar-, Äpfel-, Wein-, Zitronen-, Fumar-, Milch-, Glykol- oder Brenztraubensäure, Benzoe- oder im Phenylrest substituierte Benzoesäuren wie Toluylsäure oder p-Nitro-benzoesäure oder Salicylsäure, Furancarbon- und/oder Mandelsäure.

Bevorzugt werden Essig-, Oxal-, Wein-, Ameisen-, Zitronen-, Äpfel-, Fumar-, Dichloressig-, Trichloressig- und/oder Propionsäure angewandt.

Besonders geeignet sind Essig-, Oxal-, Wein-, Ameisen- und/oder Zitronensäure.

Als Löse- bzw. Verdünnungsmittel für diese Ausführung der Cyclisierung der Verbindung IV in Form von Salzen oder Salzgemischen sind im Prinzip alle gegenüber der Verbindung IV inerten organischen Lösemittel und/oder Wasser geeignet. Vorteilhaft werden C₁-C₄-Alkohole, Tetrahydrofuran, Dioxan, Methyl-t.-butylether, Acetonitril, N,N-Dimethylform- und/oder -acetamid, niedere Glykol-di- und -monoether, wie 1,2-Dimethoxyethan, Methyl- und/oder Ethylglykol, und/oder Methylacetat, Ethylacetat, Aceton, Chloroform, Dichlormethan, Ameisensäure und/oder Essigsäure verwendet.

Eine bevorzugte Ausführungsform des erfindungsgemäßen Verfahrens besteht darin, daß die Verbindung IV in Gegenwart von Eisessig und/oder Ameisensäure, mit oder ohne Zusatz einer oder mehrerer mittelstarker bis starker Säuren 0,3 bis 5 Stunden auf eine Temperatur von 25°C, vorzugsweise 45°C, bis 120°C, vorzugsweise 95°C, erwärmt wird.
Eisessig und/oder Ameisensäure dienen dabei zugleich als Lösemittel und werden, bezogen auf die Verbindung IV, in einem stöchiometrischen Überschuß angewandt. Von den gegebenenfalls mitzuverwendenden mittelstarken bis starken Säuren seien Oxal-, Citracon-, Wein-, Zitronen-, Trifluoressig-, Cyanessig-, Chloressig-, Dichloressig- und/oder Trichloressigsäure und/oder Polyphosphorsäure beispielhaft genannt.

Im Bedarfsfall wird die Verbindung I aus dem isolierten Salz in für solche Basenbildungen allgemein bekannter Weise freigesetzt und gegebenenfalls durch Zusatz einer entsprechenden Säure in ein (anderes) in Wasser lösliches oder leicht lösliches Salz übergeführt.

Die im Zusammenhang mit der Salzbildung der Verbindung I genannten "Säureäquivalente" beziehen sich auf die Verbindung I als einsäurige Base.

Das erfindungsgemäße Verfahren zur Herstellung der Verbindung I wird durch nachstehendes Formelschema erläutert.

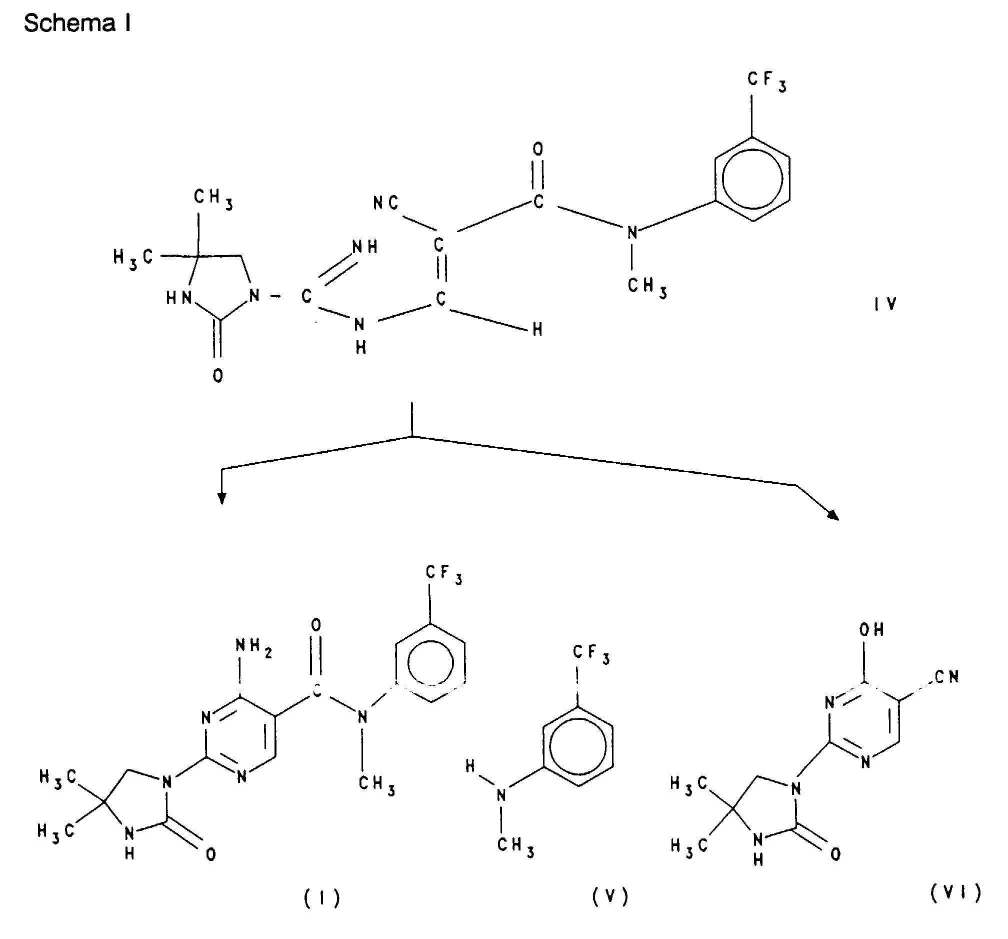

Das bei der Cyclisierung der Verbindung IV als Nebenprodukt gebildete 3-Methylamino-benzotrifluorid V kann isoliert und in den Herstellungsprozeß von III zurückgeführt werden.

Das Hydrochlorid der Verbindung der Formel I besitzt wertvolle pharmakologische Eigenschaften und kann daher als Arzneimittel verwendet werden. Es zeichnet sich beispielsweise durch hypolipidämische Eigenschaften aus.

Es wurde überraschenderweise gefunden, daß das in Wasser leicht lösliche Hydrochlorid der Verbindung der Formel I den LDL-Rezeptor-Level erhöht. Das Hydrochlorid der Verbindung I ist daher zur Behandlung von Lipidstoffwechselstörungen, die durch eine Stimulierung des hepatischen LDL-Rezeptors günstig beeinflußt werden können, geeignet. Die Erfindung betrifft daher auch die Verwendung vom Hydrochlorid der Verbindung I zur Behandlung der genannten Lipidstoffwechselstörungen.

Die Wirkung beruht auf einer raschen direkten Stimulation des LDL-Rezeptors (apoB/E Rezeptor) in der Leber. Über die Stimulation des natürlichen Cholesterin-Katabolismuswegs senkt das Hydrochlorid der Verbindung I das atherogene LDL und VLDL. Die HMG-CoA Reduktase Inhibitoren oder Ionenaustauscherharze hingegen stimulieren durch Absenkung der intrazellulären Cholesterinspiegel bzw. durch Erhöhung des Cholesterinbedarfs für die Biosynthese von Gallensäuren indirekt die Expression der LDL-Rezeptoren auf der Leberzelle.Somit sind Nebenwirkungen, wie sie bei den Statinen bedingt durch ihre drastische Inhibition der HMG-CoA Reduktase auch in anderen Organen als der Leber zu beobachten sind (z.B. Beeinflussung der Ubichinonbildung) nicht zu erwarten. Solche Nebenwirkungen wurden bislang auch nicht beobachtet. Compliance-Probleme wie bei den Ionenaustauscherharzen, die durch die erforderlichen großen täglichen Dosen von 15 bis 30 g hervorgerufen werden, sind nicht zu befürchten.

Besonders gefährlich bei der Atherogenese sind bestimmte cholesterin-transportierende Lipoproteinfraktionen wie die kleinpartikulären LDL-Fraktionen. Der Katabolismus des überwiegenden Teils des LDL (75 %) erfolgt durch den LDL-Rezeptor in der Leber. In den Leberzellen wird das Cholesterin dann teilweise zu Gallensäuren metabolisiert und in Form von Galle exkretiert, teilweise erfolgt auch eine direkte Ausscheidung von Cholesterin über die Galle. Es ist somit im Prinzip bereits anerkannt und bereits therapeutisch durch die indirekt wirksamen Substanzen (wie Statine) belegt, daß der LDL-Rezeptor ein idealer Angriffspunkt für ein hypolipidämisch wirksames Antiatherosklerotikum beim Menschen ist.

Das Hydrochlorid der Verbindung I erhöht den LDL-Rezeptor-Level. Die erhöhte LDL- (IDL-, Chylomikronen Remnant-)Aufnahme in die Leberzellen, bedingt durch die so erhöhten LDL-Rezeptor-Level auf der Zelloberfläche, führt dann zu einem verstärkten hepatischen Abbau des LDL-Cholesterins. Die HDL-Spiegel werden nicht beeinflußt.

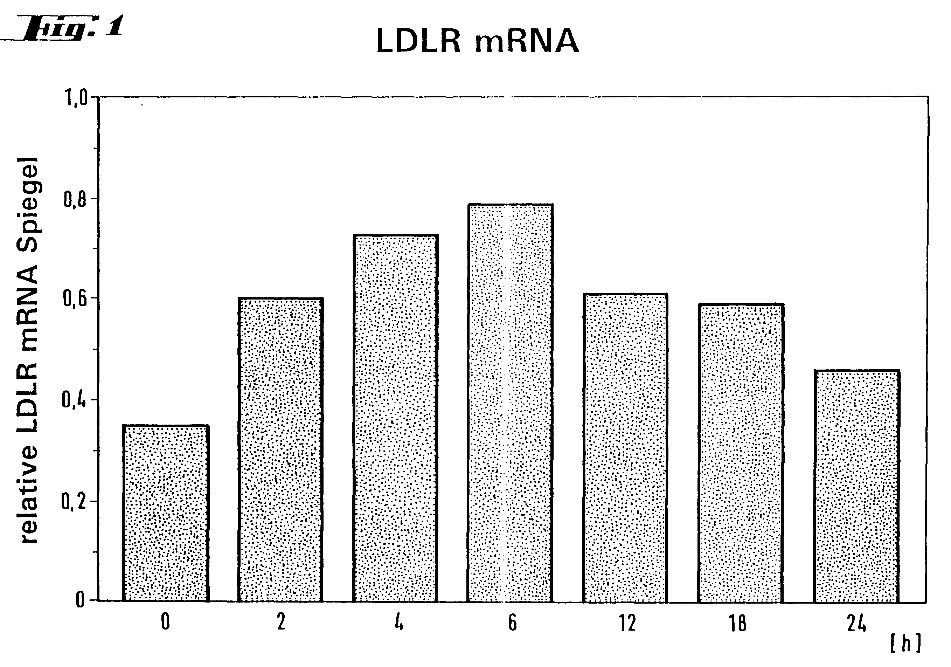

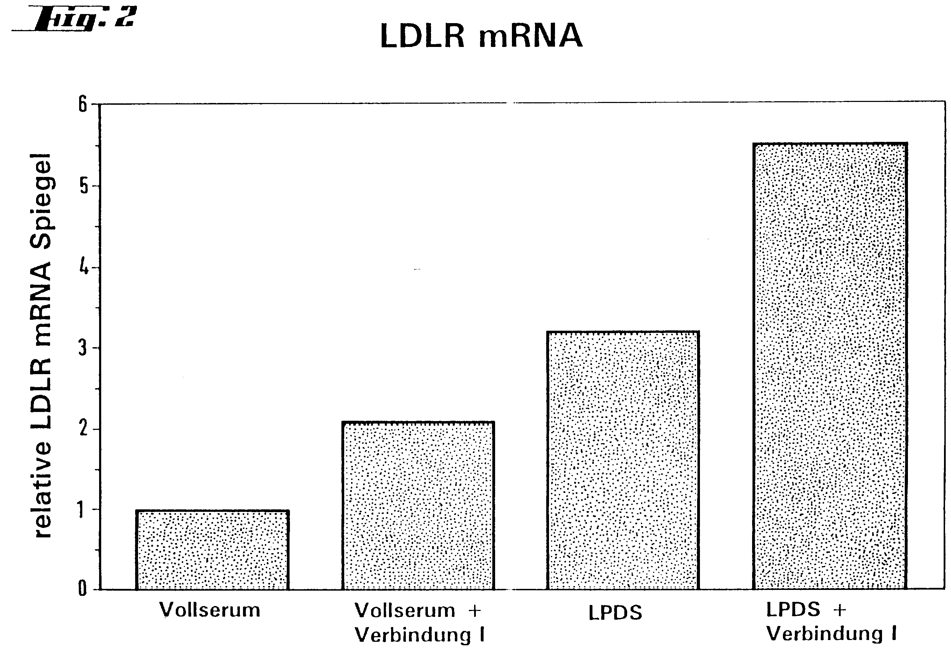

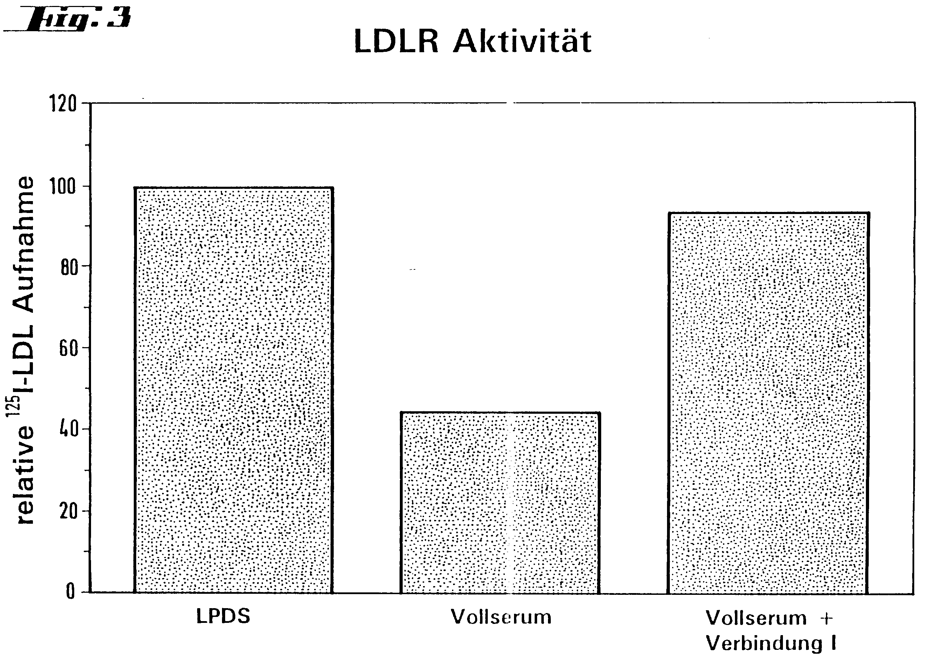

Das Hydrochlorid der Verbindung der Formel I stellt somit ein ideales antiatherosklerotisch wirksames Prinzip dar. Folgende Befunde belegen den aufgefundenen Wirkmechanismus, der die beschriebenen Verbindungen als besonders geeignet für die Behandlung solcher Hyperlipidämien, die auf einer verminderten LDL-Rezeptor-Aktivität beruhen, erscheinen läßt:
1. In der humanen Hepatozytom-Zellinie HepG2 sowie in Rattenlebern werden die LDL-Rezeptor-mRNA Spiegel innerhalb weniger Stunden nach Applikation des Hydrochlorids der Verbindung I um das 2 bis 2,5-fache erhöht (Figur 1). Die LDL-Rezeptor-mRNA-Stimulierung ist unabhängig von der Cholesterin "Versorgungslage", wie durch Inkubation der Zellinie in Vollserum und in Lipoprotein-defizientem (LDL-defizientem) Serum gezeigt werden konnte (Figur 2). Diese Beobachtungen wurden bei der Durchführung folgender Tests gemacht:
Die Präparation der mRNA wurde nach der von Chomczynski, P. und Sacchi, N. in Anal. Biochem. 162, 156 (1987) beschriebenen Methode durchgeführt. Bei Organen (wie z.B. Leber) wurde das tiefgefrorene Gewebe auf Trockeneis zuvor im Mörser homogenisiert und die mRNA mittels Oligo dT nach Standardmethoden weiter angereichert (vgl. Sambrook, J., Fritsch, E. F. und Maniatis, T., Molecular Cloning, zweite Auflage, Cold Spring Harbor (1989); in dieser Methodensammlung finden sich auch Beschreibungen aller weiteren hier verwendeten relevanten molekularbiologischen Standardverfahren). 5 bis 20 µm der so gewonnenen gelösten mRNA wurden nach Standardverfahren denaturiert und auf 1%igen horizontalen Agarosegelen aufgetrennt. Die mRNA wurde mittels Kapillarblot auf Hybond N Membranen (Amersham) transferiert. Als spezifische Hybridisierungsprobe diente ein partieller LDL-Rezeptor cDNA Klon und als interner Standard ein Plasmid, das ein β-Aktin-Gen enthielt. Beide Plasmide wurden mittels eines random Primer Kit von Amersham bis zu einer spezifischen Aktivität von 5 x 10⁹ cpm/µg markiert. Vorhybridisierung, Hybridisierung und Waschen der Filter erfolgten nach Standardverfahren. Die Filter wurden anschließend auf Cronex 4 Filmen (Dupont) über Nacht bis zu 14 Tagen in Gegenwart einer Verstärkerfolie bei -70°C exponiert und die Hybridisierungssignale über die Filmschwärzungsintensität mit einem handelsüblichen Laserdensiometer quantifiziert. Anschließend wurde der Quotient aus der Intensität der LDL-Rezeptorbande und der Aktinbande als internem Standard zur Korrektur von Ausbeutevariationen bestimmt.
Figur 1 zeigt die Stimulierung der LDL-Rezeptor-mRNA Expression in vivo in Rattenlebern in Abhängigkeit von der Zeit nach Applikation. Lebergewebe wurde nach einmaliger oraler Gabe von 30 mg/kg des Hydrochlorids von Verbindung I zu den gegebenen Zeitpunkten entnommen und in flüssigem Stickstoff schockgefroren. Anschließend wurde die mRNA wie vorstehend beschrieben präpariert und mittels der Northern-Blot-Technik die relativen LDL-Rezeptor-mRNA-Level bestimmt. Nach 6 Stunden ist der LDL-Rezeptor-mRNA-Level um das etwa 2,5-fache erhöht.
Figur 2 zeigt die Stimulierung der LDL-Rezeptor-mRNA-Expression auf HepG2 Zellen in Vollserum und in Lipoprotein-defizientem Serum. Die HepG2 Zellen wurden zuvor in MEM Standardmedium, welches 10 %iges fötales Kälberserum mit und ohne Hydrochlorid der Verbindung I (10⁻⁶ M) enthielt, 16 Stunden inkubiert. Trotz der Vorstimulierung durch das delipidierte Medium wird die LDL-Rezeptor-mRNA von der beschriebenen Verbindung weiter um den gleichen Faktor überinduziert, wie er bei der Stimulierung in Vollserum beobachtet wurde. Lipoprotein-defizientes Serum (LPDS) wurde dabei mittels Ultrazentrifugation nach einem Standardverfahren (Goldstein, J.L., Basu, S.K. und Brown, M.S., Methods Enzymol. 98, 241 (1983)) hergestellt. Die Präparation der mRNA im Serum erfolgte analog der oben beschriebenen Methode.
2. Die erhöhte mRNA Synthese führt auch zu einer entsprechenden Erhöhung funktionaler LDL-Rezeptorspiegel auf HepG2 Zellen. Die Aufnahme von ¹²⁵J markiertem homologen LDL erhöht sich bis auf das zweieinhalbfache (Figur 3).
In diesem Test wurde homologes nach Standardverfahren gereinigtes LDL nach der Jod-Monochlorid Methode mit ¹²⁵J markiert. Verwendet wurde dabei die literaturbekannte Methode von Bilheimer, die eine Modifikation der ursprünglich von McFarlaine entwickelten Jodierungstechnik darstellt (vgl. Bilheimer, D.W., Eisenberg, S., und Levy, R.L., Biochem. Biophys. Acta, BBA 260, 212 (1972)).
Zur Bestimmung der ¹²⁵J LDL Inkorporation wurden HepG2 Zellen 36 Stunden mit und ohne Hydrochlorid der Verbindung I vorinkubiert und anschließend über 3 Stunden mit jodmarkiertem LDL bei 37°C im Inkubator inkubiert. Anschließend wurden die Zellen in Gegenwart von 2 % Rinderserumalbumin gewaschen, die Zellen mit 1 ml 0,1 N NaOH lysiert und die inkorporierte Radioaktivität gemessen. Über eine Proteinbestimmung mit der Lowry-Methode wurde dann, wie allgemein üblich, die spezifische Radioaktivitätsaufnahme (totale Radioaktivitätsaufnahme abzüglich der unspezifischen Radioaktivitätsaufnahme) pro mg Protein berechnet und die aufgenommene Radioaktivität des Kontrollserums als 100 % gesetzt.
3. Bei der HMG-CoA-Reduktase-Bestimmung in vitro an teilgereinigter HMG-CoA-Reduktase aus Rattenleber (vgl. Avigan J., Bathena, S.J., und Schreiner, M.E., J. Lipid Res. 16, 151 (1975) und Philippi, B.W., und Shapiro, D.J., J. Lipid Res. 20, 588 (1979)) beeinflußt das Hydrochlorid von Verbindung I bei 10⁻⁵ M Zusatz gegenüber Kontrollinkubationen die Enzymaktivität nicht (Streuung 10 %), Lovastatin-Na (mit geöffnetem Lactonring) hat in diesem Versuch eine IC₅₀ bei 3 x 10⁻⁹ M.
4. In vivo spiegelt sich die erhöhte LDL-Rezeptoraktivität bei Versuchstieren in einer Absenkung der durch den LDL-Rezeptor (apoB/E Rezeptor) metabolisierbaren Lipoproteinfraktionen wieder, d.h. es wird eine Senkung der LDL- und VLDL-Spiegel beobachtet. Die folgenden Tests wurden durchgeführt:
4.1 Wirkung auf Serumlipoproteine normolipämischer männlicher Ratten im subchronischen Versuch
Gruppen von jeweils 10 männlichen Ratten des Stammes HOE: WISKf (SPF 71) mit einem Ausgangsgewicht über 180 g, erhielten täglich einmal (morgens) das Prüfpräparat bzw. das Vergleichspräparat in 1 % wäßriger ®Tylose MH 300-Lösung per Magensonde (0,5 ml/100 g Körpergewicht); die jeweilige Kontrollgruppe erhielt nur das Vehikel. Die letzte (7.) Applikation erfolgte 24 Stunden vor Blutentnahme und Tötung. Zu Futter und Wasser bestand freier Zugang während des Versuches. 24 Stunden vor der Blutentnahme wurde das Futter entzogen.
Zur Analyse der Serum-Lipopoteine wurde das Serum aller 10 Ratten einer Gruppe gepoolt. Die Serum-Lipoproteine wurden mit der präparativen Ultrazentrifuge (KONTRON TGA 65, Rotor BECKMAN 50.4 Ti) getrennt.
Folgende Bedingungen gemäß Koga, S., Horwitz, D.L., und Scanu, A.M., Journal of Lipid Research 10, 577 (1969) und Havel, R.J., Eder, H.A., und Bragdon, H.H., J. Clin. Invest. 34, 1345 (1955) wurden für die Trennung von VLDL, LDL und HDL verwendet:

| | | | |
|---|---|---|---|
| 1. | VLDL: | Dichte < 1,006, | 16 Stunden bei 40 000 Upm |
| 2. | LDL: | Dichte 1,006 - 1,04, | 16 Stunden bei 40 000 Upm |
| 3. | HDL: | Dichte 1,04 - 1,21, | 18 Stunden bei 40 000 Upm |

Zur enzymatischen Bestimmung des Cholesterins und der Triglyceride in den getrennten Lipoproteinfraktionen wurden Testkombinationen von BOEHRINGER/Mannheim (vgl. Siedel, J., Schlumberger, H., Klose, S., Ziegenhorn, J., und Wahlefeld, A.W., J. Clin. Chem. Clin. Biochem. 19, 838 (1981) und Wahlefeld, A.W., in: H. O. Bergmeier: Methoden der enzymatischen Analyse, 2. Auflage, Band II, Verlag Chemie 1974, S. 1878) verwendet. Die Proteine wurden nach Lowry (Lowry, O. H., Roseborough, N. J., Farr, A.L., und R. J. Randell, J. Biol. Chem. 193, 265 (1951)) bestimmt.
Die Ergebnisse sind in Tabelle 3 zusammengefaßt. Als Vergleichspräparat diente Clofibrat.

**Tabelle 3**

| Verminderung der atherogenen Serumlipoproteine männlicher Wistarratten nach 7-tägiger oraler Behandlung | | | | | |
|---|---|---|---|---|---|
| % Veränderung gegen Kontrolle | | | | | |
| | Dosis mg/kg/Tag | TOTAL-CHOLESTERIN | PROTEIN | | TRIGLYCERIDE |
| Verbindung | | LDL | VLDL | LDL | VLDL |
| Verbindung I* | 30 | -64 | -59 | -52 | -79 |
| Verbindung I* | 10 | -49 | -52 | -41 | -62 |
| Verbindung I* | 3 | -32 | -24 | -25 | -25 |
| Verbindung I* | 1 | -17 | + 4 | -15 | + 8 |
| Clofibrat | 100 | -31 | -30 | -32 | -34 |

| | | | | | |
|---|---|---|---|---|---|
| * als Hydrochlorid | | | | | |

4.2 Wirkung auf die Hypercholesterinämie der männlichen Ratte
Gruppen aus je 10 männlichen Ratten des Stammes HOE: WISKf (SPF 71) mit einem Gewicht von ca. 200 g erhielten täglich morgens einen Cocktail, der die zur Erzeugung einer kombiniert diätetisch-hormonellen Hypercholesterinämie benötigten Stoffe sowie jeweils das zur Hemmung der Hypercholesterinämie zu prüfende Präparat in entsprechender Dosierung enthielt. Der Cocktail setzte sich aus dem Gemisch von 100 g Cholesterin, 30 g Propylthiouracil und 100 g Cholsäure in 1 I Erdnußöl zusammen. Per Schlundsonde wurden davon 1 ml/100 g Körpergewicht verabreicht. Die zu prüfenden Präparate wurden in einer solchen Konzentration in diesem Cocktail suspendiert, daß bei Verabfolgung der vorerwähnten Menge die in der Tabelle 4 angegebene Tagesdosis enthalten war. Das sonstige Vorgehen der Behandlung und die Analyse der einzelnen Parameter erfolgten gemäß den unter 4.1 erwähnten Angaben.
Als Vergleichpräparat diente Clofibrat.
Die Ergebnisse sind in Tabelle 4 zusammengefaßt, wobei unter a) die Absolutwerte und unter b) die prozentualen Veränderungen angegeben sind. 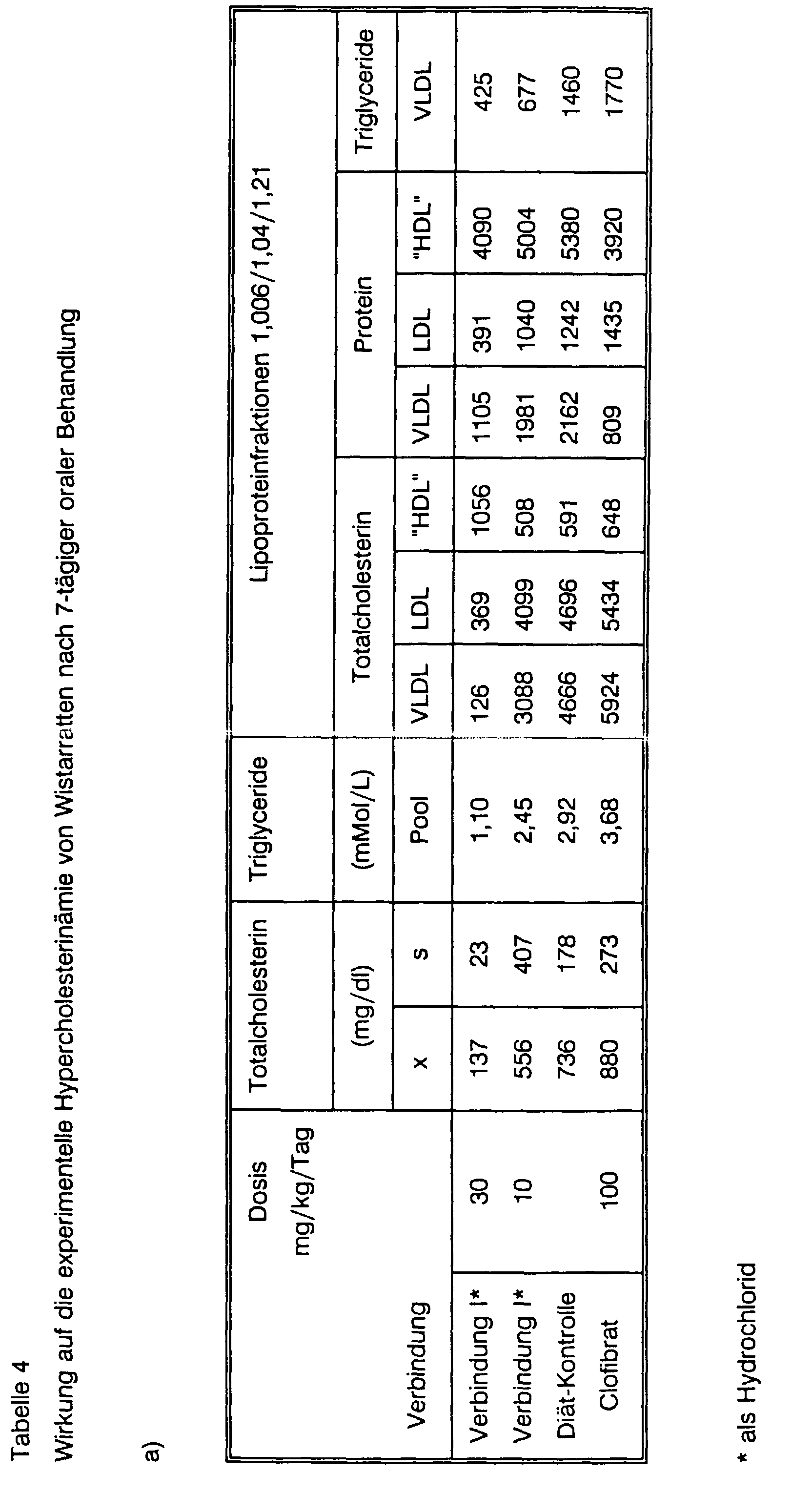 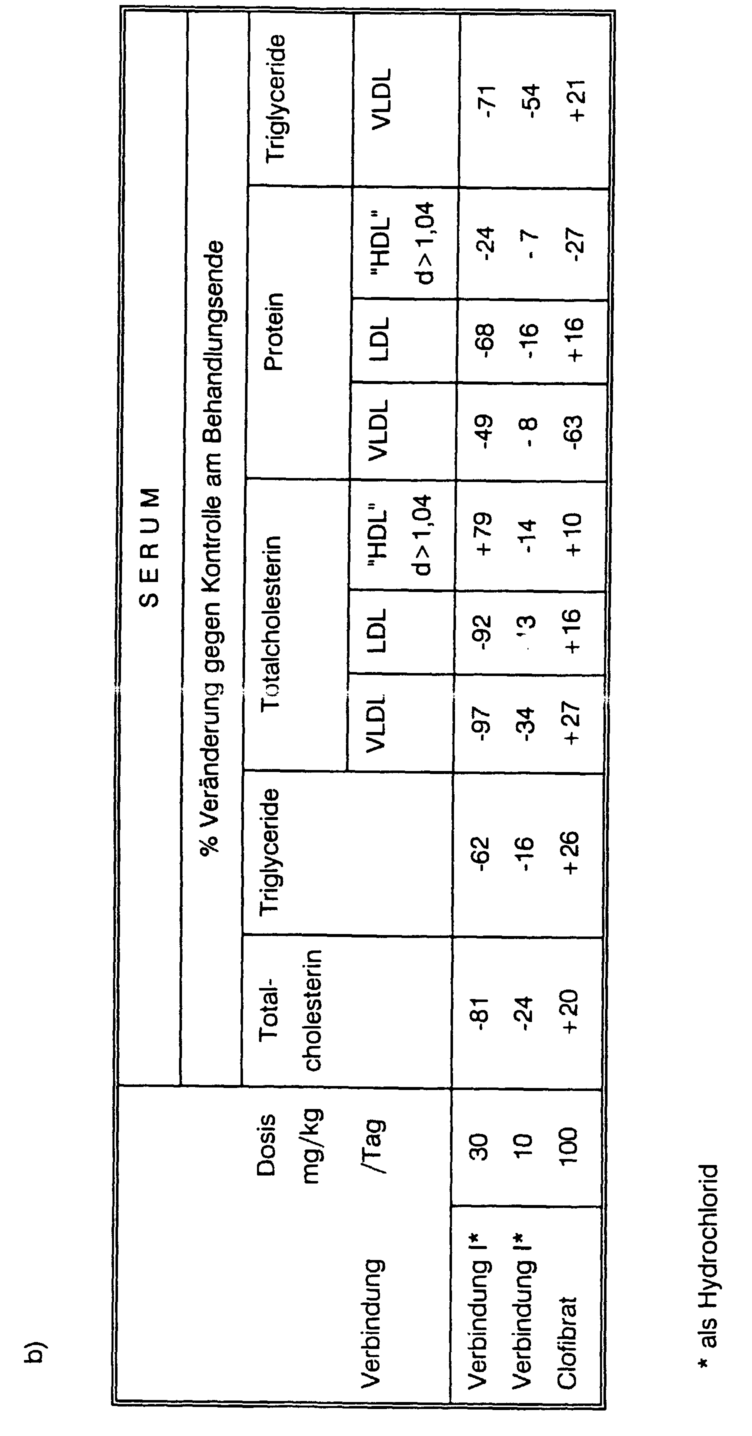
4.3 Wirkung auf die kohlenhydratinduzierte Hypertriglyceridämie
Gruppen aus je 10 männlichen Sprague-Dawley-Ratten (von Züchter Mollegard im Gewicht von 250 - 280 g) erhielten an 4 aufeinanderfolgenden Tagen je eine Applikation der in PEG 400 gelösten Verbindung per Schlundsonde. Zusätzlich nach der 3. Applikation, sowie 8 und 24 Stunden später, erhielten sie 2,5 g Fruktose/kg, ebenfalls per Schlundsonde. Außerdem wurde ihnen nach der 3. Präparatapplikation 50 %ige Fruktoselösung als Trinkwasser ad libitum angeboten sowie das Futter entzogen. 3 Stunden nach der letzten Präparat/Fruktose-Applikation erfolgte die retroorbitale Blutentnahme, woraus das Serum für die Triglyceridbestimmung bzw. VLDL-Präparation in der Ultrazentrifuge (Flotation bei d = 1,006) gewonnen wurde. 1 Woche vor der 1. Applikation bis zur 3. Applikation erhielten die Tiere als Futter eine Diät mit 40 % Casein im Futtermehl.
Aufgrund dieser Behandlung entsteht bei den Tieren eine Hypertriglyceridämie, die dem menschlichen Fredrickson Phenotypus IV in etwa entspricht. Die Serum-Triglyceride der Normaltiere erhöhten sich von 0,54 auf 1,1 mMol/L bzw. die VLDL-Triglyceride von 0,67 auf 1,81. Wie in der Tabelle 5 gezeigt, vermag das Hydrochlorid der Verbindung I den Anstieg der VLDL-Triglyceride noch mit 1 mg/kg/Tag um nahezu die Hälfte zu hemmen. Clofibrat in der 100-fach höheren Dosis führt nur zu einer geringen Hemmwirkung.
Die Ergebnisse sind in Tabelle 5 zusammengefaßt.

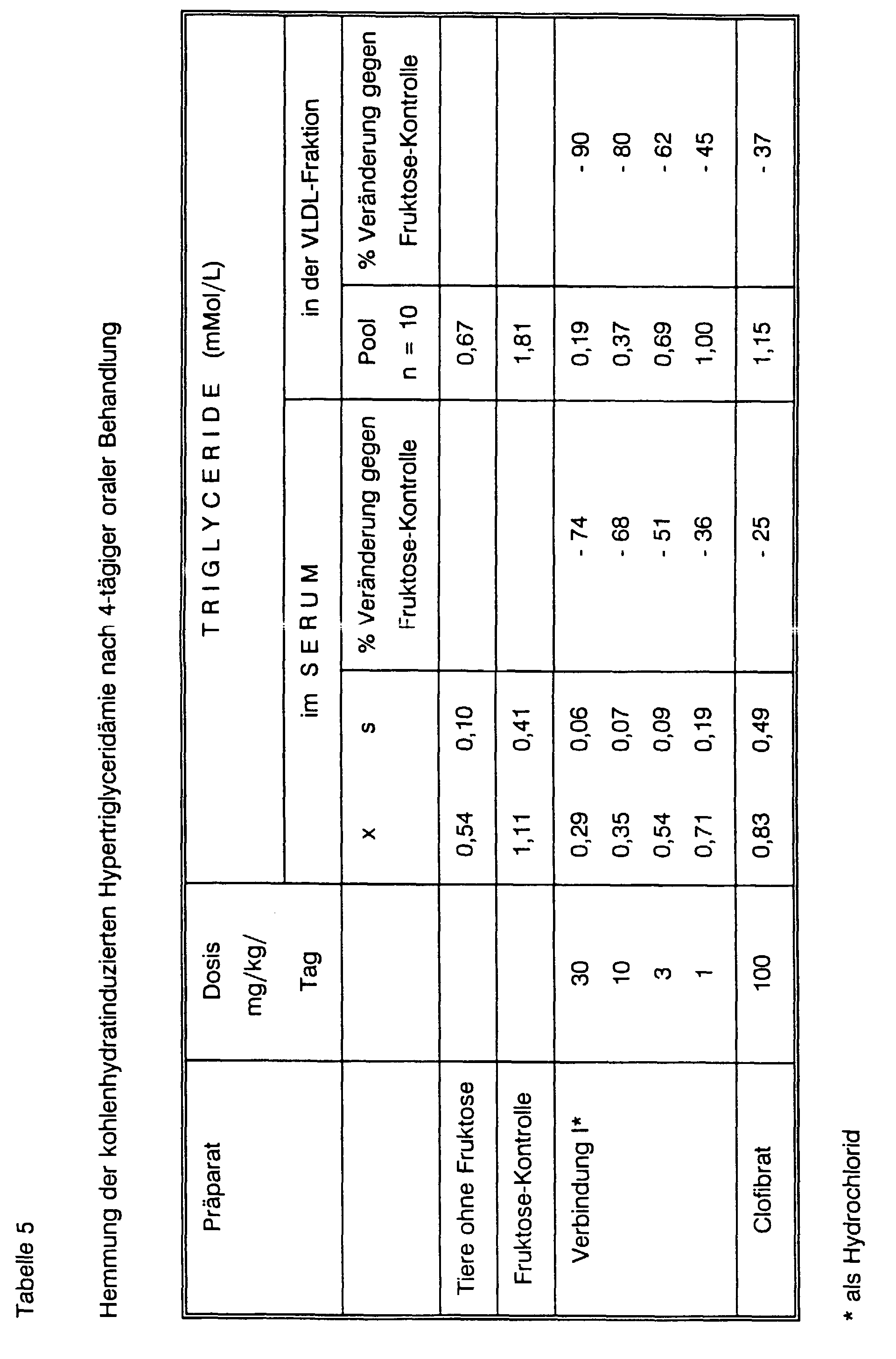

Das Hydrochlorid der Verbindung der Formel I ist ferner aufgrund ihrer reduzierten Hemmwirkung auf die Zellproliferation dem NH-Analogen von Verbindung I, dem 4-Amino-2-(4,4-dimethyl-imidazolidin-2-on-1-yl)-pyrimidin-5-carbonsäure-[N-(3-trifluormethyl-phenyl)-amid] (HOE 402, Imanixil) bzw. dem Hydrochlorid dieser Verbindung deutlich überlegen, wie der nachfolgend beschriebene Test und die Testergebnisse zeigen:
Die reduzierte Hemmwirkung auf die Zellproliferation wurde am exponentiell wachsenden tierischen (Mausleukämie-Zellen, L 1210) bzw. humanen (Bronchialkarzinom-Zellen, A 549; Kolonkarzinom-Zellen, HT 29) Tumorzellen geprüft. Dabei wurden die Tumorzellen in RPMI-Standard-Medium 72 Stunden mit verschiedenen Konzentrationen der Testsubstanzen inkubiert. Als Kontrolle dienten Zellinkubationen im lediglich frischen Medium. Es wurden 4-fach Inkubationen angesetzt. Nach 65 Stunden wurde MTT (3-(4,5-Dimethyl-2-thiazolyl)-2,5-diphenyl-2H-tetrazolium-bromid) hinzugesetzt, welches durch unversehrte Zellen zu einem roten unlöslichen Formazanfarbstoff reduziert wird. Nach 7 bis 24 Stunden weiterer Inkubation wurde der Zellüberstand vorsichtig entfernt und der Formazanfarbstoff in den Zellen durch Zugabe von DMSO solubilisiert und photometrisch bei 492 nm quantitativ gemessen. Ausgewertet wurde das Verhältnis der Extinktion der Substanz-lnkubationen zu der Kontroll-Inkubation. Der Variationskoeffizient jeder Inkubationsserie war geringer als 15 %. In der folgenden Tabelle 6 sind die IC₅₀-Werte (IC₅₀ [µg/ml] ist die Konzentration der Prüfsubstanz pro ml, die für eine 50 %ige Hemmung erforderlich ist) aus diesen Untersuchungen angegeben. Daraus ist ersichtlich, daß das Hydrochlorid der Verbindung I in einer wesentlich höheren Dosis gegeben werden muß, um die gleiche proliferationshemmende Wirkung zu haben (IC₅₀) wie Imanixil-Hydrochlorid.

**Tabelle 6**

| | MTT-ASSAY IC₅₀ [µg/ml] | | |
|---|---|---|---|
| Substanz | L1210 | HT 29 | A 549 |
| Imanixil-Hydrochlorid | 0,7 | 4,3 | 3,4 |
| Verbindung I als Hydrochlorid | 13,4 | > 100 | > 100 |

Das Hydrochlorid der Verbindung I besitzt gegenüber HOE 402 bzw. HOE 402-Hydrochlorid eine deutlich reduzierte Proliferationshemmung, die den Sicherheitsabstand bei einer massiven iatrogenen Überdosierung mit möglicher reversibler Leukozyten-Reduzierung deutlich verbessert.

Das Hydrochlorid der Verbindung der Formel I kann aufgrund seiner pharmazeutischen Eigenschaften als Arzneimittel Verwendung finden, wobei man es entweder allein oder vermischt mit geeigneten Trägerstoffen und/oder verträglichen Verdünnungsmitteln und gegebenenfalls auch noch mit anderen Zusätzen verabreicht.

Die vorliegende Erfindung betrifft auch pharmazeutische Zubereitungen, die neben nicht toxischen, inerten pharmazeutisch geeigneten Trägerstoffen erfindungsgemäßen Wirkstoff enthalten oder die aus dem erfindungsgemäßen Wirkstoff bestehen sowie Verfahren zur Herstellung dieser Zubereitungen.

Unter nicht toxischen, inerten pharmazeutischen geeigneten Trägerstoffen sind pharmazeutisch unbedenkliche, feste, halbfeste oder flüssige Verdünnungsmittel, Füllstoffe und Formulierungshilfsmittel jeder Art zu verstehen, die nach dem Vermischen mit dem Wirkstoff diesen in eine für die Verabreichung geeignete Form bringen.

Als geeignete Anwendungsformen der Verbindung 1 bzw. des in Wasser leicht löslichen Monohydrochlorids dieser Verbindung kommen beispielsweise Tabletten, Dragees, Pulver, Kapseln, Pillen, wäßrige Lösungen, Suspensionen und Emulsionen, gegebenenfalls sterile injizierbare Lösungen, nichtwäßrige Emulsionen, Suspensionen und Lösungen, Sprays sowie Zubereitungsformen mit protrahierter Wirkstoff-Freigabe in Betracht.

Die therapeutisch wirksamen Verbindungen sollen in den oben aufgeführten pharmazeutischen Zubereitungen zweckmäßig in einer Konzentration von etwa 0,1, vorzugsweise von 0,5, bis 99,0, vorzugsweise bis 70,0, Gewichtsprozent der Gesamtmischung vorhanden sein.

Die Anwendungskonzentrationen für Lösungen sowie Aerosole in Form von Spray betragen im allgemeinen von 0,1 bis 20, vorzugsweise 0,5 - 5, Gewichtsprozent.

Die oben aufgeführten pharmazeutischen Zubereitungen können außer dem genannten Wirkstoff auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen. In den Beispielen 27 und 28 sind geeignete Tablettenzusammensetzungen beschrieben.

Zur vorliegenden Erfindung gehört auch die Verwendung von erfindungsgemäßen Wirkstoffen sowie von pharmazeutischen Zubereitungen, die den erfindungsgemäßen Wirkstoff enthalten, in der Humanmedizin zur Verhütung, Besserung und/oder Heilung der oben angeführten Erkrankungen.

Der Wirkstoff oder die pharmazeutischen Zubereitungen können oral, parenteral, intraperitoneal und/oder rectal appliziert werden.

Die z.B. als Hypolipidämikum verwendbare Verbindung der vorliegenden Erfindung kann zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Kapseln (Gelatinekapseln), welche den Wirkstoff zusammen mit Verdünnungsmitteln bzw. Trägerstoffen, z.B. Lactose, Dextrose, Rohrzucker, Mannitol, Sorbitol, Cellulose, verschiedenen Stärkearten und/oder Glycin, und Gleitmitteln wie Kieselerde, Talk, Stearinsäure oder deren Salze, wie Magnesium- oder Calciumstearat, und/oder Polyethylenglykol enthalten. Tabletten enthalten zweckmäßigerweise ebenfalls Bindemittel wie Magnesiumcarbonat, Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Pärparate, die gegebenenfalls weitere pharmakologisch wirksame Stoffe enthalten können, werden z.B. mittels konventioneller Misch-Granulier- und Dragierverfahren hergestellt und enthalten 0,1 % bis vorzugsweise etwa 80 %, bevorzugt etwa 0,5 % bis etwa 65 % des Wirkstoffs.

Die orale Anwendung erfolgt in pharmazeutisch üblichen Zubereitungen, beispielsweise in Form von Tabletten, Dragees oder Kapseln, die z.B. pro Tagesdosis 5, vorzugsweise 20, bis 1000 mg, vorzugsweise bis 200 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die nachfolgenden Beispiele dienen zur näheren Erläuterung der Erfindung.

Die in den nachfolgenden Beispielen angegebenen Schmelz- bzw. Zersetzungspunkte sind unkorrigiert.

Dünnschichtchromatographien wurden auf DC-Fertigplatten, Kieselgel 60, F-254 der Firma Riedel-de Haen AG mit den in den Beispielen jeweils angegebenen Fließmitteln ausgeführt.

### Beispiel 1

Zu einer Suspension von 6,25 g (40 mMol) 1-Amidino-4,4-dimethyl-2-oxoimidazolidin in 120 ml wasserfreiem 1,2-Dimethoxyethan wurden unter Rühren bei 20°C 11,93 g (40 mMol) 2-Cyano-3-ethoxy-acrylsäure-[N-methyl-N-(3-trifluormethylphenyl)-amid] (Verbindung III) gegeben. Nach ca. 5 Min. Rühren war eine Lösung entstanden, aus der, beginnend nach ca. 12 Min., ein kristalliner Stoff ausfiel. Man rührte 2 Stunden bei 20 - 24°C weiter, saugte anschließend den Feststoff ab und wusch mit Ether nach. Das Filtrat wurde im Vakuum bei < 26°C eingedampft. Den kristallinen Rückstand (8,1 g) suspendierte man in 25 ml Ether und saugte nach 20 Min. den Feststoff ab. Beide erhaltenen kristallinen Anteile wurden zusammen in ca. 200 ml Wasser suspendiert, 40 Min. bei Raumtemperatur gerührt, abgesaugt, mit etwas Wasser nachgewaschen und 48 Stunden bei 20 - 25°C im Vakuum über P₂O₅ getrocknet. Man erhielt auf diese Weise 14,02 g (= 85,8 % Ausbeute) 1-Cyano-1-[N-methyl-N-(3-trifluormethyl-phenyl)-carbamoyl]-2-[(imino-(4,4-dimethyl-2-oxo-imidazolidin-1-yl)-methyl)-amino]-ethen (Verbindung IV), Schmelz- bzw. Zersetzungspunkt: 186 - 189°C. Diese Verbindung zeigt im IR-Spektrum eine charakteristische CN-Bande bei 2212 cm⁻¹. Das erhaltene Produkt ist laut DC (CH₂Cl₂/C₂H₅OH:10:1) nahezu rein (R_{F} 0,44 ± 0,01). Laut ¹H-NMR-Spektrum liegt einheitlich ein Stereoisomeres (E oder Z) vor.

| C₁₈H₁₉F₃N₆O₂ (408.40) | | | | |
|---|---|---|---|---|
| berechnet: | C 52,94 | H 4,69 | F 13,96 | N 20,58 % |
| gefunden: | C 52,70 | H 4,65 | F 13,20 | N 20,30 % |

### Beispiel 2

4,69 g (30 mMol) 1-Amidino-4,4-dimethyl-2-oxo-imidazolidin wurden bei 70°C in einer Mischung aus 38 ml 1,2-Dimethoxyethan und 45 ml Isopropanol gelöst. Diese Lösung wurde auf 20°C abgekühlt und bei 20°C mit 9,0 g (30,2 mMol) 2-Cyano-3-ethoxy-acrylsäure-[N-methyl-N-(3-trifluormethyl-phenyl)-amid] versetzt. Man rührte 1 Stunde bei 20 - 24°C nach, saugte den ausgefallenen kristallinen Niederschlag ab und wusch ihn mit Ether nach. Das Filtrat wurde wie im Beispiel 1 beschrieben bearbeitet. Dabei sind weitere 0,74 g kristallines Produkt erhalten worden. Dieser und der vorher direkt isolierte Hauptteil wurden vereinigt, wie im Beispiel 1 beschrieben mit Wasser ausgerührt, wiederum abgesaugt und getrocknet. Man erhielt 10,76 g (= 87,8 % Ausbeute) Produkt, das laut DC fast reine Verbindung IV darstellt.

### Beispiel 3

Zu einer Lösung von 3,12 g (20 mMol) 1-Amidino-4,4-dimethyl-2-oxo-imidazolidin in einer Mischung aus 20 ml Tetrahydrofuran und 10 ml Methanol gab man bei 18 - 20°C 5,97 g (20 mMol) 2-Cyano-3-ethoxy-acrylsäure-[N-methyl-N-(3-trifluormethyl-phenyl)-amid] und rührte 1,5 Stunden bei 20°C nach. Danach wurde der ausgefallene Feststoff abgesaugt. Dieser und das anfallende Filtrat wurden analog wie im Beispiel 1 weiter aufgearbeitet. Man erhielt 6,67 g (= 81,7 % Ausbeute) Produkt, das laut DC nahezu reine Verbindung IV darstellt.

### Beispiel 4

Zu einer Mischung aus 70 ml 1-Propanol und 5,5 ml einer 30 %igen Lösung von Natriummethylat in Methanol (= 30 mMol) gab man bei Raumtemperatur 7,12 g (30 mMol) 1-Amidino-4,4-dimethyl-2-oxo-imidazolidin-hydrobromid und rührte 20 Min. bei Raumtemperatur nach. Anschließend gab man bei 20°C 8,95 g (30 mMol) 2-Cyano-3-ethoxy-acrylsäure-[N-methyl-N-(3-trifluormethyl-phenyl)-amid] zu und rührte 30 Min. bei 20°C und 1,5 Stunden bei 20 - 25°C nach. Danach wurde der ausgefallene Feststoff abgesaugt, mit 1-Propanol und mit Wasser gewaschen und wie im Beispiel 1 beschrieben getrocknet. Das ursprüngliche Filtrat wurde wie im Beispiel 1 aufgearbeitet. Man erhielt 6,82 g (= 55,7 % Ausbeute) laut DC nahezu reine Verbindung IV.

### Beispiel 5

4,74 g (20 mMol) 1-Amidino-4,4-dimethyl-2-oxo-imidazolidin-hydrobromid wurden in 47 ml siedendem 1-Propanol gelöst und bei 88 - 90°C mit 3,60 g (= 3,67 ml, 20 mMol) einer 30 %igen Lösung von Natriummethylat in Methanol versetzt. Die entstandene Suspension wurde auf 45°C abgekühlt, bei 45°C 30 Min gerührt, dann auf 20°C abgekühlt, bei 20°C mit 5,97 g (20 mMol) 2-Cyano-3-ethoxyacrylsäure-[N-methyl-N-(3-trifluormethyl-phenyl)-amid] versetzt und 1,8 Stunden bei Raumtemperatur nachgerührt. Der ausgefallene Feststoff wurde nach Verdünnen der breiigen Masse mit 50 ml Ether abgesaugt und mit Ether nachgewaschen. Dieses Produkt (Primärkristallisat) und der Rückstand des ursprünglichen Filtrats (Mutterlauge) wurden wie im Beispiel 1 beschrieben weiter aufgearbeitet und getrocknet. Man erhielt 6,23 g (= 76,3 % Ausbeute) laut DC nahezu reine Verbindung IV.

### Beispiel 6

Eine Lösung von 4,74 g (20 mMol) 1-Amidino-4,4-dimethyl-2-oxo-imidazolidin-hydrobromid in 47 ml Methanol wurde bei Raumtemperatur mit 3,60 g (= 3,67 ml, 20 mMol) 30 %iger NaOCH₃/CH₃OH-Lösung versetzt und 30 Min. bei 45°C gerührt. Dann kühlte man die Lösung auf 20°C ab, gab bei 20°C 5,97 g (20 mMol) 2-Cyano-3-ethoxy-acrylsäure-[N-methyl-N-(3-trifluormethyl-phenyl)-amid] zu und rührte 20 Min. bei 20°C, 1,5 Stunden bei Raumtemperatur und 30 Min. unter Eisbadkühlung nach. Der ausgefallene Feststoff wurde abgesaugt, mit Ether nachgewaschen und weiter wie im Beispiel 1 beschrieben aufgearbeitet. Ebenso wurde der Rückstand vom Filtrat (Mutterlauge) wie im Beispiel 1 beschrieben weiter aufgearbeitet und getrocknet. Man erhielt 6,35 g (= 77,7 % Ausbeute) laut DC (CH₂Cl₂/C₂H₅OH = 10:1) nahezu reine Verbindung IV.

In analoger Weise kann anstelle des hier verwandten Hydrobromids das Hydrochlorid des 1-Amidino-4,4-dimethyl-2-oxo-imidazolidins (20 mMol = 3,85 g) eingesetzt werden.

### Beispiel 7

Zu einer Suspension von 8,17 g (20 mMol) 1-Cyano-1-[N-methyl-N-(3-trifluormethylphenyl)-carbamoyl]-2-[(imino-(4,4-dimethyl-2-oxo-imidazolidin-1-yl)-methyl)-amino]-ethen (Verbindung IV) in 48 ml Ethanol tropfte man bei Raumtemperatur 3,1 ml einer 6,6 molaren Lösung von HCI in Ether und rührte 1 Stunde bei 1 - 3°C nach. Danach saugte man den Feststoff ab, wusch ihn mit Ether nach und trocknete ihn 20 Stunden bei Raumtemperatur im Vakuum (4 - 6 mbar). Man erhielt 8,34 g (= 90,7 % Ausbeute) reines 1-Cyano-1-[N-methyl-N-(3-trifluormethyl-phenyl)-carbamoyl]-2-[imino-(4,4-dimethyl-2-oxo-imidazolidin-1-yl)-methyl)-amino]-ethen-hydrochlorid, Schmelzpunkt 180 - 181°C (≡ Umwandlungspunkt; Substanz verfestigt sich wieder);

| C₁₈H₂₀ClF₃N₆O₂ (444,86) | | | | | |
|---|---|---|---|---|---|
| berechnet: | C 48,60 | H 4,53 | Cl 7,97 | F 12,81 | N 18,89 % |
| gefunden: | C 48,20 | H 4,35 | Cl 8,0 | F 12,75 | N 18,60 % |

### Beispiel 8

Eine Suspension von 1,00 g (2,45 mMol) der Verbindung IV in 6 ml Ethylacetat wurde bei RT mit 5 ml einer 0,5 molaren Lösugn von HNO₃ in Ethylacetat versetzt und 3 Stunden unter Eisbadkühlung gerührt. Danach saugte man den Feststoff ab, wusch ihn mit Ethylacetat und Ether nach und trocknete ihn bei Raumtemperatur im Vakuum (4 bis 7 mbar) 24 Stunden. Man erhielt 1,11g ( 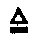 94 % Ausbeute) reines Nitrat der Verbindung IV, Schmelzpunkt 142 bis 143°C.

| C₁₈H₂₀F₃N₇O₅ (471,41) | | | | |
|---|---|---|---|---|
| berechnet: | C 45,86 | H 4,28 | F 12,09 | N 20,80 % |
| gefunden: | C 46,3 | H 4,6 | F 12,0 | N 21,1 % |

### Referenzbeispiel 9

Eine Lösung von 1,634 g (4 mMol) 1-Cyano-1-[N-methyl-N-(3-trifluormethyl-phenyl)-carbamoyl]-2-[(imino-(4,4-dimethyl-2-oxo-imidazolidin-1-yl)-methyl)-amino]-ethen (Verbindung IV) in 3 ml Ameisensäure (98 - 100 %ig) wurde 2 Stunden auf 50°C erwärmt. Danach zog man im Vakuum die Ameisensäure ab, nahm den Rückstand in Dichlormethan auf, gab 20 ml Wasser zu und stellte mit verdünnter Natronlauge einen pH zwischen 9 und 10 ein. Die organische Phase wurde abgetrennt. Die wäßrige Phase schüttelte man noch zweimal mit Dichlormethan aus. Die vereinigten Dichlormethan-Extrakte wurden nacheinander mit 2 ml 0,5 N NaOH und zweimal mit Wasser ausgewaschen, mit MgSO₄ getrocknet und im Vakuum eingedampft. Den verbliebenen festen Rückstand vermischte man mit Ether/Heptan 1:1 und saugte ihn ab. Nach dem Trocknen (vgl. Beispiel 13) erhielt man 0,93 g (= 56,9 % Ausbeute) reines 4-Amino-2-(4,4-dimethyl-2-oxo-imidazolidin-1-yl)-pyrimidin-5-carbonsäure-[N-methyl-N-(3-trifluormethyl-phenyl)-amid], Schmelzpunkt 210 - 211°C; DC in CH₂Cl₂/C₂H₅OH = 10:1.

| C₁₈H₁₉F₃N₆O₂ (408,40) (Verbindung I) | | | | |
|---|---|---|---|---|
| berechnet: | C 52,94 | H 4,69 | F 13,96 | N 20,58 % |
| gefunden: | C 52,8 | H 5,0 | F 13,4 | N 20,4 % |

### Referenzbeispiel 10

Eine 3 Stunden auf 40°C erwärmte Lösung von 3,268 g (8 mMol) der Verbindung IV in 6 ml Ameisensäure wurde wie im Beispiel 9 beschrieben aufgearbeitet. Dabei sind 2,03 g (= 62,1 % Ausbeute) Verbindung I, Schmelzpunkt 210 - 211°C, erhalten worden.

### Referenzbeispiel 11

Eine Suspension von 1,634 g (4 mMol) der Verbindung IV in 3 ml Eisessig wurde bei 50°C gerührt. Nach 1,5 Stunden war eine Lösung entstanden, die noch 1 Stunde bei 50°C gerührt, danach bei Raumtemperatur mit 25 ml Wasser verdünnt und mit 5,5 ml 32%iger Natronlauge auf pH 9 gestellt wurde. Man extrahierte die erhaltene Mischung mehrmals mit Ethylacetat. Die vereinigten Ethylacetat-Extrakte wurden zweimal mit etwas Wasser ausgewaschen, mit Na₂SO₄ getrocknet, filtriert und im Vakuum eingedampft. Den Rückstand löste man in 100 ml CH₂Cl₂. Diese Lösung wurde nacheinander viermal mit je 10 ml 0,5 N NaOH und zweimal mit je 4 ml Wasser ausgeschüttelt, mit Na₂SO₄ getrocknet, filtriert und im Vakuum eingedampft. Den Rückstand mischte man mit ca. 10 ml Ether bei 2 - 5°C an und saugte ihn ab. Nach dem Trocknen (wie im Beispiel 13) erhielt man 1,26 g (= 77,1 % Ausbeute) an reiner Verbindung I, Schmelzpunkt 210,5 - 211,5°C.

### Referenzbeispiel 12

Eine Mischung aus 2,45 g (6 mMol) der Verbindung IV, 24 ml Tetrahydrofuran (THF) und 2,70 g (45 mMol) Eisessig wurde unter Rückflußkochen gerührt, wobei nach ca. 10 Min. eine Lösung entstand, die 3,8 Stunden am Rückfluß gekocht und danach im Vakuum eingedampft wurde. Der verbliebene Rückstand wurde mit 8 ml Wasser versetzt und mit 2N Salzsäure auf pH 1 gestellt. Der dabei ausgefallene Feststoff wurde abgesaugt, mit Ether gewaschen und getrocknet. Bei diesem Feststoff (0,68 g) handelte es sich um die als Nebenprodukt gebildete Verbindung VI. Das saure wäßrige Filtrat wurde mit verdünnter Natronlauge auf pH 9 - 10 eingestellt, wobei sich ein öliges Produkt abschied, das bei Zusatz von einigen ml Ether kristallisierte. Nach 1 Stunde Kühlung auf 2 - 4°C wurde das Kristallisat abgesaugt, mit wenig Ether nachgewaschen und, wie im Beispiel 13 beschrieben, getrocknet. Man erhielt 1,19 g (= 48,6 % Ausbeute) an reiner Verbindung I, Schmelzpunkt 210 - 211°C.

### Referenzbeispiel 13

Eine Lösung von 2,45 g (6 mMol) der Verbindung IV und 0,66 g (7,33 mMol) wasserfreier Oxalsäure in 36 ml absolutem THF wurde 8 Stunden am Rückfluß gekocht, anschließend bei ca. 30°C mit 34,5 ml CH₂Cl₂ versetzt und 17 Stunden bei RT stehengelassen. Die ausgefallene kristalline Substanz wurde abgesaugt, mit etwas CH₂Cl₂ nachgewaschen und bei 100°C im Vakuum (3 - 7 mbar) getrocknet. Dabei erhielt man 1,36 g Produkt, das mit Oxalsäure verunreinigtes Hydrogenoxalat der Verbindung I darstellt. Dieses Produkt wurde in 5 ml Wasser gelöst, mit 2,3 ml 2N NaOH versetzt und 1 Stunde unter Eiskühlung gerührt. Der dabei ausgefallene kristalline Niederschlag wurde abgesaugt, mit Wasser und Ether gewaschen und 17 h bei 100°C im Vakuum (180 mbar) getrocknet. Man erhielt 0,98 g (= 40 % Ausbeute) der Verbindung I, Schmelzpunkt 209 - 210°C.

### Referenzbeispiel 14

Zu einer Mischung von 20 ml Methanol und 0,392 g (~ 4 mMol) konzentrierter Schwefelsäure gab man bei Raumtemperatur 1,634 g (4 mMol) de Verbindung IV und kochte die entstandene Lösung 9,5 Stunden am Rückfluß. Danach wurde im Vakuum eingedampft. Den verbliebenen Rückstand versetzte man mit ca. 10 ml Wasser und stellte mit verdünnter Natronlauge auf pH 9 ein. Die Mischung wurde mehrmals mit Ether extrahiert. Die gesammelten Etherauszüge wurden mit wenig Wasser ausgewaschen, mit Na₂SO₄ getrocknet, filtriert und im Vakuum eingedampft. Der verbliebene Rückstand wurde mit Heptan/Ether 1:1 verrieben. Das entstandene Kristallisat saugte man ab und trocknete es wie im Beispiel 13 beschrieben. Man erhielt 0,22 g (= 13,5 % Ausbeute) reine Verbindung I, Schmelzpunkt 210 - 211°C.

### Referenzbeispiel 15

Eine Mischung von 2,45 g (6 mMol) der Verbindung IV, 180 mg (2 mMol) Oxalsäure und 5 ml Eisessig wurde unter Rühren auf 50°C erwärmt, wobei nach 30 Minuten eine Lösung entstanden war. Man rührte 2,5 Stunden weiter bei 50°C und zog dann den Eisessig im Vakuum ab. Den Rückstand versetzte man mit 30 ml CH₂Cl₂ und 20 ml Wasser, stellte die wäßrige Phase mit verdünnter NaOH auf pH 9 - 10, trennte die Phasen und extrahierte die wäßrige noch dreimal mit CH₂Cl₂. Die vereinigten CH₂Cl₂-Extrakte wurden mit Wasser ausgewaschen, über MgSO₄ getrocknet, filtriert und im Vakuum eingedampft. Der verbliebene Rückstand wurde mit Ether/Heptan 1:1 vermischt. Das Kristallisat wurde abgesaugt, mit Ether/Heptan 3:1 nachgewaschen und im Vakuum (4 - 6 mbar) getrocknet. Man erhielt 1,83 g (= 74,7 % Ausbeute) praktisch reine Verbindung I, Schmelzpunkt 209 - 210°C.

### Referenzbeispiel 16

Eine Suspension von 2,45 g (6 mMol) der Verbindung IV in einer Mischung aus 4,60 ml Eisessig und 0,40 ml Wasser wurde bei 50°C gerührt. Nach 30' lag eine Lösung vor, die weitere 2,5 Stunden bei 50°C gerührt und anschließend im Vakuum eingedampft wurde. Zum Rückstand gab man ca. 10 ml Wasser, stellte mit verdünnter NaOH auf pH 9-10 ein und extrahierte diese Mischung mehrmals mit CH₂Cl₂. Der CH₂Cl₂-Extrakt wurde zweimal mit wenig Wasser ausgewaschen, über Na₂SO₄ getrocknet, filtriert und im Vakuum eingedampft. Den kristallinen Rückstand verrührte man mit ca. 10 ml Ether. Nach 1 Stunde Kühlung im Eisbad saugte man den Feststoff ab, wusch ihn mit Ether nach und trocknete ihn gemäß Beispiel 13. Man erhielt 1,74 g (= 71 % Ausbeute) reine Verbindung I,
Schmelzpunkt 210 - 211°C.

### Referenzbeispiel 17

Eine Mischung aus 2,45 g (6 mMol) Verbindung IV, 820 mg (5 mMol) Trichloressigsäure und 4,25 ml Eisessig wurde 2 Stunden bei 70°C gerührt und anschließend im Vakuum eingedampft. Der verbliebene Rückstand wurde in 100 ml Ethylacetat aufgenommen und nacheinander je zweimal mit je 20 ml 2N NaOH und je 10 ml Wasser ausgeschüttelt. Nach Trocknen über MgSO₄ wurde die Lösung filtriert und im Vakuum eingedampft. Der Rückstand wurde in 25 ml Ether gelöst, woraufhin ein Kristallisat ausfiel. Man rührte 30 Minuten bei Raumtemperatur nach und dampfte die Mischung im Vakuum ein. Der kristalline Rückstand wurde in Diisopropylether suspendiert, abgesaugt und 15 Stunden bei 100°C im Vakuum getrocknet. Man erhielt 1,36 g (= 55,5 % Ausbeute) reines 4-Amino-2-(4,4-dimethyl-2-oxo-imidazolidin-1-yl)-pyrimidin-5-carbonsäure-[N-methyl-N-(3-trifluormethylphenyl)-amid], Schmp. 209 - 210°C.

### Referenzbeispiel 18

Eine Suspension von 1,78 g (4 mMol) 1-Cyano-1-[N-methyl-N-(3-trifluormethyl-phenyl)-carbamoyl]-2-[(imino-(4,4-dimethyl-2-oxo-imidazolidin-1-yl)-methyl)-amino]-ethen-hydrochlorid in 20 ml Wasser wurde bei 80°C gerührt. Nach 1,5 Stunden lag eine klare wäßrige Phase vor, aus der sich eine verklebte Masse als Klumpen abgeschieden hatte. Nach weiteren 5,9 Stunden Rühren bei 80°C wurde mit verdünnter Salzsäure auf pH 1 gestellt und filtriert. Das Filtrat wurde mit NaOH auf pH 9 - 10 eingestellt, woraufhin kristalliner Niederschlag ausfiel. Nachdem man 1 Stunde unter Eiskühlung nachrührte, wurde der Feststoff abgesaugt, mit Wasser und Ether nachgewaschen und gemäß Beispiel 13 getrocknet. Man erhielt 0,47 g (= 28,8 % Ausbeute) der Verbindung I, Schmelzpunkt 209 - 210°C.

### Referenzbeispiel 19

10,74 g (26,3 mMol) 1-Cyano-1-[N-methyl-N-(3-trifluormethyl-phenyl)-carbamoyl]-2-[(imino-(4,4-dimethyl-2-oxo-imidazolidin-1-yl)-methyl)-amino]-ethen (Verbindung IV) wurden in einen, auf 190°C vorbeheizten Kolben gegeben, der mit einem Destillationsaufsatz verschlossen wurde. Unter magnetischer Rührung beließ man die Substanz 5 Minuten bei 190°C und 6 Min bei 190 - 192°C. Danach wurde die Apparatur evakuiert und das als Nebenprodukt gebildete 3-Methylaminobenzotrifluorid unter Vakuum im Verlauf von 10 Min. bei einer Badtemperatur von 189°C - 175°C destillativ weitgehend entfernt. Die entstandene braune, geschmolzene Masse wurde in 100 ml CH₂Cl₂ aufgenommen und diese Lösung wurde nacheinander dreimal mit je 35 ml 0,5 N NaOH, zweimal mit je 20 ml Wasser und viermal mit je 25 ml 1N Salzsäure ausgeschüttelt. Die gesammelten salzsauren wäßrigen Extrakte wurden mit Aktivkohle geklärt, filtriert und mit Natronlauge auf pH 9 eingestellt. Der dabei ausfallende Niederschlag wurde in CH₂Cl₂ aufgenommen und die wäßrige Phase wurde einmal mit CH₂Cl₂ extrahiert. Die vereinigten CH₂Cl₂-Extrakte wurden mit Wasser ausgewaschen, über MgSO₄ getrocknet, filtriert und im Vakuum eingedampft. Der verbleibende, amorphe Rückstand wurde in Ether gelöst, woraufhin in einigen Minuten kristalline Substanz ausfiel. Diese saugte man nach einigen Stunden ab und trocknete sie bei 100°C 15 Stunden im Vakuum (180 mbar). Man erhielt auf diese Weise 3,83 g (= 35,7 % Ausbeute) DC reines 4-Amino-2-(4,4-dimethyl-2-oxo-imidazolidin-1-yl)-pyrimidin-5-carbonsäure-[N-methyl-N-(3-trifluormethyl-phenyl)-amid] (Verbindung I),
Schmelzpunkt 209 - 210°C.

| C₁₈H₁₉F₃N₆O₂ (408,40) | | | | |
|---|---|---|---|---|
| berechnet: | C 52,94 | H 4,69 | F 13,96 | N 20,58 % |
| gefunden: | C 52,8 | H 5,0 | F 13,35 | N 20,4 % |

DC in CH₂Cl₂/C₂H₅OH = 10:1.

In dem bei der Aufarbeitung erhaltenen wäßrig-alkalischen Extrakt befindet sich die als Nebenprodukt gebildete Verbindung VI. Die gute Wasserlöslichkeit des Hydrochlorids der Verbindung I ermöglicht die beschriebene Art der Aufarbeitung, die eine bequeme und effektive Abtrennung von Nebenprodukten bei der Gewinnung der Verbindung I beinhaltet.

### Referenzbeispiel 20

2,67 g (6 mMol) 1-Cyano-1-[N-methyl-N-(3-trifluormethyl-phenyl)-carbamoyl]-2-[(imino-(4,4-dimethyl-2-oxo-imidazolidin-1-yl)-methyl)-amino]-ethen-hydrochlorid werden unter Vakuum (4 - 6 mbar) in einem auf 190°C vorgeheizten Bad 10 Min. auf 187-189°C erwärmt. Nach dem Erkalten wurde die erstarrte Schmelze mit 5 ml 2N Natronlauge vermischt. Man setzte 12 ml Diethylether/Diisopropylether 5:1 zu. Unter Rühren bildeten sich zunächst zwei klare Phasen aus denen ein Kristallisat ausfiel. Man rührte 1 Stunde unter Eiskühlung, saugte das Kristallisat ab, wusch es mit Wasser nach und nahm es in 50 ml CH₂Cl₂ auf. Die gebildete Lösung wurde nacheinander dreimal mit je 5 ml 0,5 N NaOH und zweimal mit je 5 ml Wasser ausgeschüttelt, über Na₂SO₄ getrocknet, filtriert und eingedampft. Der Rückstand wurde mit Diisopropylether verrieben, abgesaugt und gemäß Beispiel 13 getrocknet. Man erhielt 0,82 g (= 32,6 % Ausbeute) der Verbindung I, Schmelzpunkt 209 - 210°C.

### Beispiel 21

Zu einer Suspension von 45,5 g (111,4 mMol) 4-Amino-2-(4,4-dimethyl-2-oxoimidazolidin-1-yl)-pyrimidin-5-carbonsäure-[N-methyl-N-(3-trifluormethyl-phenyl)-amid] (Verbindung I) in 810 ml 1,2-Dimethoxyethan tropfte man unter Rühren bei Raumtemperatur 4N Salzsäure bis der pH-Wert sich bei 3,0 hielt. Dafür wurden 28,4 ml 4N Salzsäure benötigt. Es entstand eine Lösung, die nach 30 Min. Nachrühren (bei Raumtemperatur) im Vakuum eingedampft wurde. Der verbleibende zähölige Rückstand wurde in 150 ml siedendem Aceton gelöst, woraufhin sogleich Bildung von kristallinem Niederschlag einsetzte. Nach 30 Min. Kühlung im Eisbad wurde das Kristallisat abgesaugt, mit Aceton und Ether nachgewaschen und 17 Stunden bei 100°C im Vakuum (180 mbar) getrocknet. Man erhielt 46,7 g (= 94,2 % Ausbeute) reines 4-Amino-2-(4,4-dimethyl-2-oxoimidazolidin-1-yl)-pyrimidin-5-carbonsäure-[N-methyl-N-(3-trifluormethyl-phenyl)-amid]-hydrochlorid, Schmelzpunkt 210 - 211°C.

| C₁₈H₂₀ClF₃N₆O₂ (444,86) | | | | | |
|---|---|---|---|---|---|
| berechnet: | C 48,60 | H 4,53 | Cl 7,97 | F 12,81 | N 18,89 % |
| gefunden: | C 48,4 | H 4,4 | Cl 7,8 | F 12,0 | N 18,8 % |

Die Löslichkeit dieses Hydrochlorids der Verbindung I in Wasser ist in der Tabelle 2 (Nr. 1) aufgeführt.

### Beispiel 22

Zu einer Lösung von 3,68 g (9 mMol) 2-Amino-2-(4,4-dimethyl-2-oxo-imidazolidin-1-yl)-pyrimidin-5-carbonsäure-[N-methyl-N-(3-trifluormethyl-phenyl)-amid] in 20 ml Aceton tropfte man bei Raumtemperatur 1,5 ml einer 6,6 molaren Lösung von HCI in Ether, rührte die entstandene Suspension 30 Min unter Eisbadkühlung nach und saugte den kristallinen Feststoff ab. Dieser wurde wie im Beispiel 13 beschrieben getrocknet. Man erhielt 3,75 g (93,7 % Ausbeute) reines 4-Amino-2-(4,4-dimethyl-2-oxo-imidazolidin-1-yl)-pyrimidin-5-carbonsäure-[N-methyl-N-(3-trifluormethyl-phenyl)-amid]-hydrochlorid, Schmelzpunkt 210 - 211°C.

### Beispiel 23

In analoger Weise wie im Beispiel 22 beschrieben wurde eine Lösung von 8 mMol der Verbindung I in 25 ml Ethylacetat und 5 ml Methanol mit 1,35 ml einer 6,6 molaren Lösung von HCI in Ether versetzt und anschließend im Vakuum eingedampft. Den zähöligen Rückstand löste man in 20 ml siedendem Aceton, wobei nach einigen Minuten kristalliner Niederschlag ausfiel. Nach 30 Min. Rühren unter Eisbadkühlung wurde der Feststoff abgesaugt, mit Aceton und Ether nachgewaschen und wie im Beispiel 13 beschrieben getrocknet. Man erhielt 3,28 g (92,3 % Ausbeute) reines 4-Amino-2-(4,4-dimethyl-2-oxo-imidazolidin-1-yl)-pyrimidin-5-carbonsäure-[N-methyl-N-(3-trifluormethyl-phenyl)-amid]-hydrochlorid,
Schmelzpunkt 210 - 211°C.

### Referenzbeispiel 24

Zu einer Suspension von 1,84 g (4,5 mMol) Verbindung I in 30 ml Ethylacetat gab man unter Rühren bei Raumtemperatur 9,3 ml einer 0,5 molaren Lösung von HNO₃ in Ethylacetat. Dabei entstand kurzzeitig eine Lösung aus der sogleich kristalliner Niederschlag ausfiel, der nach 2 Stunden Nachrühren bei Raumtemperatur abgesaugt und mit Ethylacetat gewaschen wurde. Nachdem das Kristallisat 17 Stunden bei 80°C im Vakuum (5 mbar) getrocknet worden war, wurden 2,02 g (95,2 % Ausbeute) reines 4-Amino-2-(4,4-dimethyl-2-oxo-imidazolidin-1-yl)-pyrimidin-5-carbonsäure-[N-methyl-N-(3-trifluormethyl-phenyl)-amid]-nitrat, Schmelzpunkt 220 - 221°C erhalten.

| C₁₈H₂₀F₃N₇O₅ (471,41) | | | | |
|---|---|---|---|---|
| berechnet: | C 45,86 | H 4,28 | F 12,09 | N 20,80 % |
| gefunden: | C 45,85 | H 4,25 | F 12,45 | N 20,90 % |

Die Löslichkeit dieses Nitrats der Verbindung I in Wasser ist in der Tabelle 2 (Nr. 2) aufgeführt.

### Referenzbeispiel 25

Zu einer Suspension von 613 mg (1,5 mMol) Verbindung I in 4 ml Acetonitril gab man bei Raumtemperatur eine Lösung von 285,5 mg (1,5 mMol) p-Toluolsulfonsäure-hydrat in 10 ml Acetonitril. Die entstandene Lösung (pH 3-4) wurde 20 Min. bei Raumtemperatur gerührt und im Vakuum eingedampft. Den Rückstand versetzte man mit Ether. Dabei kristallisierte Substanz aus, die nach 20 Min. Rühren bei Raumtemperatur abgesaugt und mit Ether gewaschen wurde. Nach dem Trocknen (8 Stunden 5-6 mbar, 80°C) erhielt man 820 mg (= 94,1 % Ausbeute) reines 4-Amino-2-(4,4-dimethyl-2-oxo-imidazolidin-1-yl)-pyrimidin-5-carbonsäure-[N-methyl-N-(3-trifluormethyl-phenyl)-amid]-p-toluolsulfonat,
Schmelzpunkt 168 - 170°C.

| C₂₅H₂₇F₃N₆O₅S (580,61) | | | | | |
|---|---|---|---|---|---|
| berechnet: | C 51,72 | H 4,68 | F 9,82 | N 14,48 | S 5,52 % |
| gefunden: | C 51,3 | H 4,7 | F 10,0 | N 14,25 | S 5,7 % |

Die Löslichkeit dieses p-Toluolsulfonats der Verbindung I in Wasser ist in der Tabelle 2 (Nr. 3) aufgeführt.

### Referenzbeispiel 26

Zu einer Suspension von 408,5 mg (1 mMol) 4-Amino-2-(4,4-dimethyl-2-oxoimidazolidin-1-yl)-pyrimidin-5-carbonsäure-[N-methyl-N-(3-trifluormethyl-phenyl)-amid] in 5 ml Acetonitril gab man bei Raumtemperatur eine Lösung von 150,5 mg (1 mMol) (R,R) (+)-Weinsäure in 3 ml Methanol, woraufhin eine Lösung entstand. Diese wurde im Vakuum bei 40°C Badtemperatur eingedampft. Der verbliebene amorphe Rückstand wurde in Ether/Acetonitril 10:1 gelöst. Nach einigen Minuten fiel Kristallisat aus. Man rührte 2 Stunden bei Raumtemperatur und 1 Stunde unter Eisbadkühlung, saugte das Kristallisat ab, wusch es mit Ether nach und trocknete es bei 85°C im Vakuum (4 - 5 mbar). Man erhielt 510 mg (= 91,3 % Ausbeute) 4-Amino-2-(4,4-dimethyl-2-oxo-imidazolidin-1-yl)-pyrimidin-5-carbonsäure-[N-methyl-N-(3-trifluormethyl-phenyl)-amid]-(R,R)-hydrogentartrat, Schmelzpunkt 152-153°C (Zersetzung).

| C₂₂H₂₅F₃N₆O₈ (558,49) | | | | |
|---|---|---|---|---|
| berechnet: | C 47,31 | H 4,51 | F 10,21 | N 15,05 % |
| gefunden: | C 46,70 | H 4,40 | F 9,65 | N 14,1 % |

Die Löslichkeit dieses Hydrogentartrats der Verbindung I in Wasser ist in der Tabelle 2 (Nr. 4) aufgeführt.

### Beispiel 27

Herstellung einer Filmtablette nachfolgend genannter Zusammensetzung:

| Bestandteile (pro Tablette) | Gewicht (mg) |
|---|---|
| 1. 4-Amino-2-(4,4-dimethyl-2-oxo-imidazolidin-1-yl)-pyrimidin-5-carbonsäure-[N-methyl-N-(3-trifluormethyl-phenyl)-amid]-hydrochlorid | 100 |
| 2. Lactose | 35 |
| 3. Maisstärke | 20 |
| 4. Mirkokristalline Cellulose | 10 |
| 5. Natrium-Stärkeglykolat | 8 |
| 6. Siliziumdioxid | 5 |
| 7. Magnesiumstearat | 2 |
| 8. Filmlack | 10 |
| Gesamtgewicht | 190 |

a) Die Substanzen 1. - 4. werden gemischt, kompaktiert und durch ein Frewitt-Sieb (Maschenweite 1,0 mm) passiert.
b) Das Granulat aus a) wird mit den Substanzen 5. - 7. bestäubt und verpreßt.
c) Die Tabletten aus b) werden mit wäßrigem Filmlack lackiert.

### Beispiel 28

Herstellung einer Filmtablette nachfolgend genannter Zusammensetzung:

| Bestandteil (pro Tablette) | Gewicht (mg) |
|---|---|
| 1. 4-Amino-2-(4,4-dimethyl-2-oxo-imidazolidin-1-yl)-pyrimidin-5-carbonsäure-[N-methyl-N-(3-trifluormethyl-phenyl)-amid]-hydrochlorid | 100 |
| 2. Lactose | 11 |
| 3. Hydroxypropylmethylcellulose | 60 |
| 4. Mikrokristalline Cellulose | 7 |
| 5. Magnesiumstearat | 2 |
| 6. Filmlack | 10 |
| Gesamtgewicht | 190 |

a) Die Substanzen 1. - 4. werden gemischt, kompaktiert und durch ein Frewitt-Sieb (Maschenweite 1,0 mm) passiert.
b) Das Granulat aus a) wird mit der Substanz 5. bestäubt und verpreßt.
c) Die Tabletten aus b) werden mit wäßrigem Filmlack lackiert.

Bei dieser Tablettenart handelt es sich um eine Zubereitung mit verzögerter Wirkstoff-Freigabe.

## Patentansprüche

1. Hydrochlorid der Verbindung der Formel I

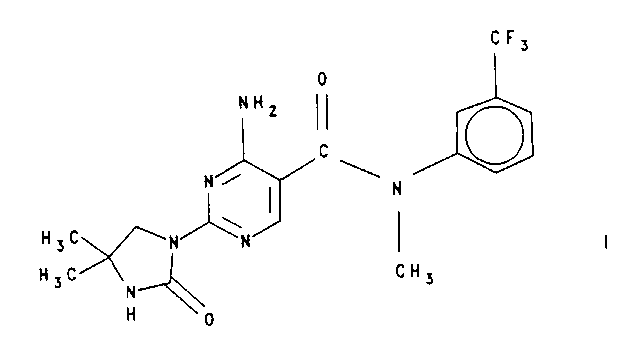

2. Arzneimittel enthaltend das Hydrochlorid der Verbindung der Formel I.

3. Verwendung des Salzes gemäß Anspruch 1 zur Herstellung eines Arzneimittels zur Behandlung von Lipidstoffwechselstörungen, die durch eine Stimulierung des hepatischen LDL-Rezeptors günstig beeinflußt werden.

4. Verwendung des Salzes gemäß Anspruch 1 zur Stimulierung des hepatischen LDL-Rezeptors.

5. Verfahren zur Herstellung des Hydrochlorids der Verbindung der Formel I, dadurch gekennzeichnet, daß man eine Verbindung der Formel IV 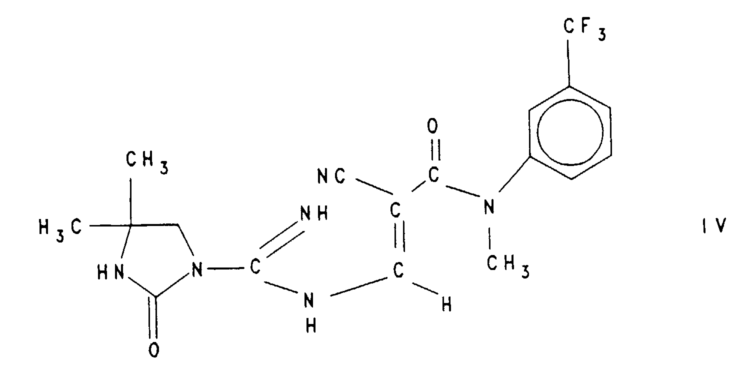 oder ein Salz oder ein Salzgemisch dieser Verbindung bei einer Temperatur von 0° bis 240°C mit oder ohne Lösemittelzusatz zu einer Verbindung der Formel I oder einem Salz oder Salzgemisch der Verbindung I cyclisiert, eine erhaltene Verbindung der Formel I in das Hydrochlorid überführt oder ein erhaltenes Salz oder Salzgemisch in das Hydrochlorid überführt.

6. Hydrochlorid der Verbindung der Formel IV

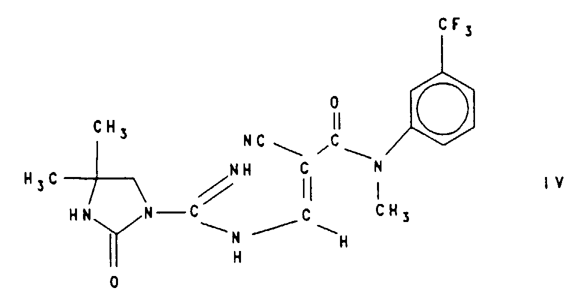

7. Verfahren zur Herstellung der Salze der Verbindung der Formel IV, gemäß Anspruch 6, dadurch gekennzeichnet, daß man eine Verbindung der Formel II 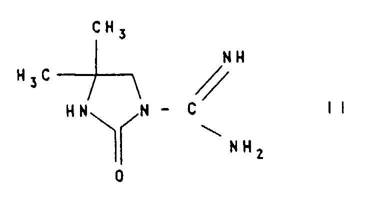 oder ein Salz dieser Verbindung mit einer Verbindung der Formel III 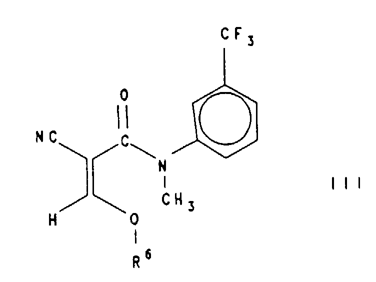 worin R⁶ C₁-C₄-Alkyl bedeutet, in Gegenwart eines organischen Löse- oder Verdünnungsmittels zu einer Verbindung der Formel IV oder einem Salz dieser Verbindung umsetzt, und die erhaltene Verbindung der Formel IV gegebenenfalls in ein Salz überführt oder ein erhaltenes Salz gegebenenfalls in die Verbindung der Formel IV überführt.

## Claims

1. The hydrochloride of the compound of the formula I

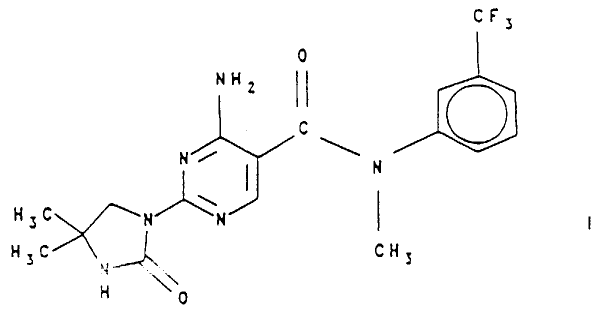

2. A pharmaceutical containing the hydrochloride of the compound of the formula I.

3. The use of the salt as claimed in claim 1 for producing a pharmaceutical for the treatment of lipid metabolism disorders which are favorably influenced by stimulation of the hepatic LDL receptor.

4. The use of the salt as claimed in claim 1 for stimulating the hepatic LDL receptor.

5. A process for the preparation of the hydrochloride of the compound of the formula I, which comprises cyclizing a compound of the formula IV 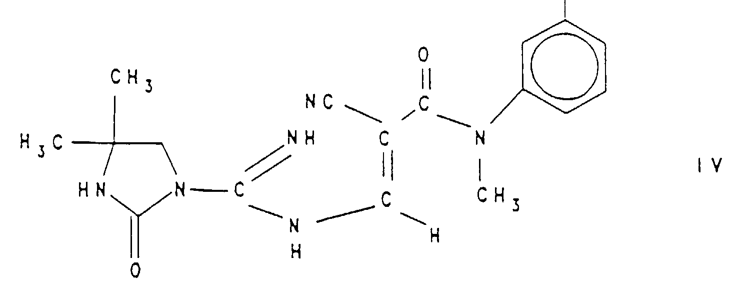 or a salt or a salt mixture of this compound at a temperature of 0° to 240°C with or without added solvent to give a compound of the formula I or salt or salt mixture of the compound I, converting a resulting compound of the formula I into the hydrochloride, or converting a resulting salt or salt mixture into the hydrochloride.

6. The hydrochloride compound of the formula IV

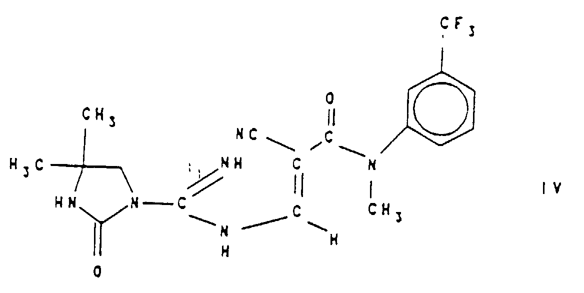

7. A process for the preparation of the salts of the compound of the formula IV, as claimed in claim 6, which comprises reacting a compound of the formula II 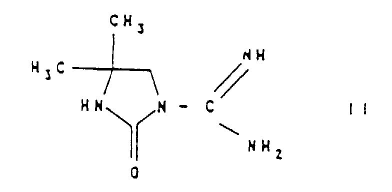 or a salt of this compound with a compound of the formula III 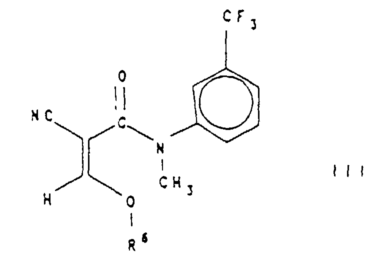 in which R⁶ is C₁-C₄-alkyl, in the presence of an organic solvent or diluent to form a compound of the formula IV or a salt of this compound, and converting the resulting compound of the formula IV where appropriate into a salt, or converting a resulting salt where appropriate into the compound of the formula IV.

## Revendications

1. Chlorhydrate du composé de formule I

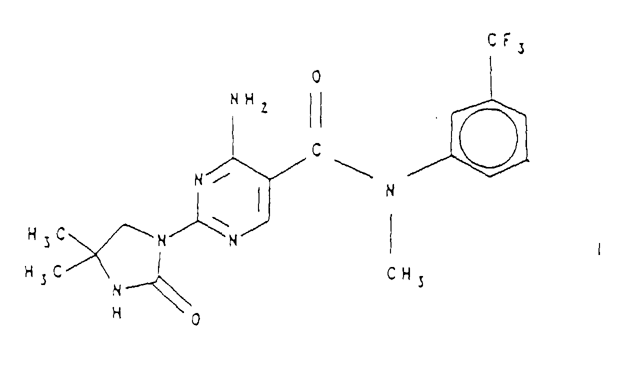

2. Médicament contenant le chlorhydrate du composé de formule I.

3. Utilisation du sel selon la revendication 1 pour la préparation d'un médicament pour le traitement des troubles du au métabolisme lipidique qui sont influencées par une stimulation du récepteur LDL hépatique.

4. Utilisation du sel selon la revendication 1 pour la stimulation du récepteur LDL hépatique.

5. Procédé de préparation du chlorhydrate du composé de formule I, caractérisé en ce que, l'on réalise la cyclisation d'un composé de formule IV 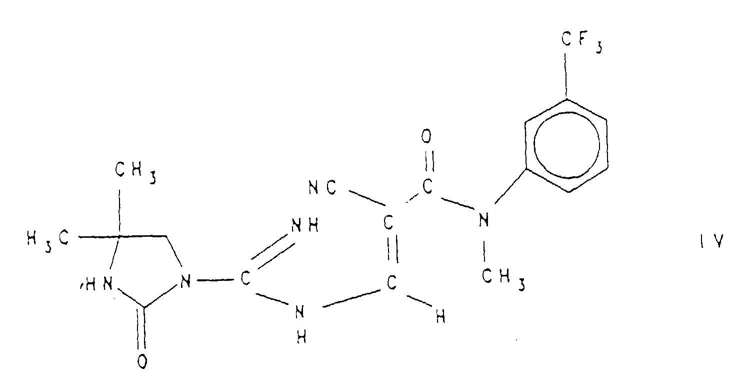 ou d'un sel ou d'un mélange de sels de ce composé à une température de 0°C à 240°C avec ou sans ajout de solvant pour obtenir un composé de formule I ou un sel ou un mélange de sels du composé I, on transforme le composé de formule I obtenu en chlorhydrate ou le sel ou le mélange de sels obtenu en chlorhydrate.

6. Chlorhydrate du composé de formule IV

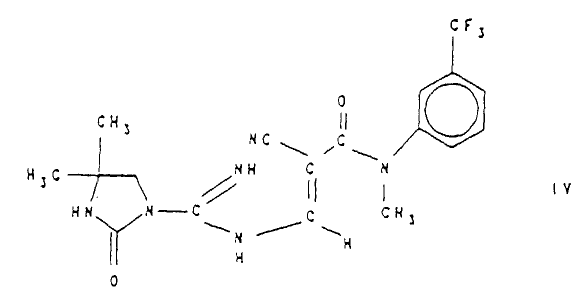

7. Procédé de préparation des sels du composé de formule IV, selon la revendication 6, caractérisé en ce que l'on fait réagir un composé de formule II 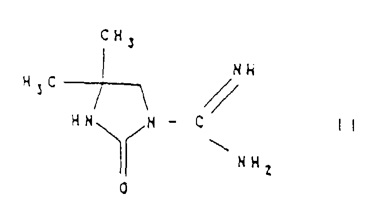 ou un sel de ce composé avec un composé de formule III 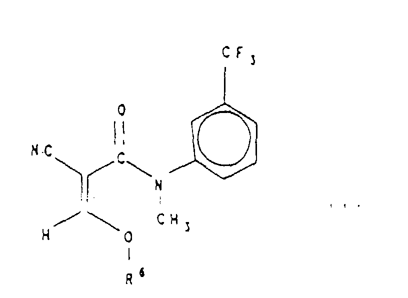 dans laquelle R⁶ représente un groupe alkyle en C₁-C₄, en présence d'un solvant ou d'un diluant organique pour former le composé de formule IV ou un sel de ce composé, et en ce que l'on transforme le composé de formule IV obtenu éventuellement en un sel ou un sel obtenu éventuellement en le composé de formule IV.
